Europäisches Patentamt

European Patent Office

Office européen des brevets

(19)

(11) Publication number: **0 279 988 B1**

## EUROPEAN PATENT SPECIFICATION

(45) Date of publication of patent specification: 24.04.91    (51) Int. Cl.⁵: **C12Q 1/00, C12Q 1/32**

(21) Application number: **87310819.5**

(22) Date of filing: **09.12.87**

(54) **Digital threshold colour control system.**

(30) Priority: **16.12.86 US 942414**
       **20.07.87 US 75817**

(43) Date of publication of application:
       **31.08.88 Bulletin 88/35**

(45) Publication of the grant of the patent:
       **24.04.91 Bulletin 91/17**

(84) Designated Contracting States:
       **AT BE CH DE ES FR GB GR IT LI LU NL SE**

(56) References cited:
       **EP-A- 0 114 267        EP-A- 0 124 909**
       **EP-A- 0 154 409        CH-A- 658 073**
       **DE-A- 3 408 062        GB-A- 2 050 601**

(73) Proprietor: **ENZYMATICS, INC.**
       **Building H Bethlehem Technology Center**
       **South Mountain Drive**
       **Bethlehem Pennsylvania 18015(US)**

(72) Inventor: **Palmer,John L.**
       **150 West Evergreen D-3**
       **Philadelphia,Pennsylvania 19118(US)**
       Inventor: **Timmerman,Marsha W.**
       **2860 Reading Road**
       **Allentown, Pennsylvania 18104(US)**

(74) Representative: **Bassett, Richard Simon et al**
       **ERIC POTTER & CLARKSON St. Mary's Court**
       **St. Mary's Gate**
       **Nottingham NG1 1LE(GB)**

## Description

### INTRODUCTION

The invention provides an improved system, devices and method for measuring qualitatively and quantitatively the concentration of NAD(P)H or NAD(P) using an enzyme, a diaphorase (lipoamide dehydrogenase), a chromogen which acts as a substrate (generally herein called a "first substrate") for the diaphorase, which generates color when reduced by NAD(P)H and a second substrate for the diaphorase, which substrate is irreversibly reduced but generates no color, at least not in the color range in which the chromogen generates color.

This invention also provides an improved system, devices and method of measuring qualitatively and quantitatively the concentration of NAD(P)H or NAD(P), without the use of diaphorase, using an enzyme, a chromogen which is directly reduced by NAD(P)H and a second substrate for the NAD(P)H, which substrate is irreversibly reduced but generates no color, at least rot in the color range in which the chromogen generates color.

### BACKGROUND OF THE INVENTION

#### 1. Field of the Invention

This invention relates to systems, more particularly a device for quantitative colorimetric analysis of organic substances, concentrations generally in biological fluids. The invention also relates to a method for making such analysis. The invention also relates to a system, a device and a method which involves the reduced and oxidized coenzymes nicotinamide-adenine dinucleotide (NADH, NAD +) or nicotinamide-adenine dinucleotide phosphate (NADPH, NADP +) (collectively herein referred to as NAD(P)H and NAD(P) +. The device may be disposable.

The invention relates to a novel and unique analog to digital colorimetric signal system, device and method which determines the concentration of NAD(P)H or of an organic substance which generates NAD(P)H in a NAD(P) + dependent dehydrogenase reaction where the dehydrogenase is specific to the substrate.

The concentration of the NAD(P)H or of the organic substance of unknown concentration, the concentration which is determined, is ascertained by an "off"-"on" change of color, preferably quite decisive and highly distinctive, like yellow to intense blue.

The invention provides a test of extreme sensitivity and accuracy coupled with great convenience. The invention has numerous applications and uses in industrial, biomedical, medical, diagnostic (e.g. in genetic engineering) and numerous other fields as will become readily apparent to one of average skill in the art to which the invention pertains.

#### 2. The Invention

The invention is operative in two different ways to generate the color corresponding to the amount of material sought to be determined: by the development of color (from colorless to color), or by the reduction of color from a high color intensity to a lower color intensity within the visible and readable range. Generically therefore, the method of the invention relates and refers to "color change".

A unique feature of the invention is that there is generated less than 1 equivalent of colored dye from the chromogen per mole of NAD(P)H.

The invention also provides for the measurement of an organic compound, generally in a biological fluid sample which in the presence of dehydrogenase is oxidized to yield NAD(P)H. Thus, the concentration of alcohol, sugar, ketones or other organic compounds can be readily measured in various concentrations without dilution, as is the conventional practice.

In accordance with the process of this invention, a competing substrate is reduced by diaphorase in the presence of NAD(P)H while concurrently the chromogen is likewise reduced so that for each mole of NAD(P)H present (or produced from another substrate) there is produced an equivalence of color less than the equivalence of NAD(P)H.

It is also in accordance with the process of this invention that a competing substrate is reduced directly by NAD(P)H while concurrently the chromogen is likewise reduced so that for each mole of NAD(P)H present or produced from another substrate there is produced an equivalence of color less than the equivalence of NAD(P)H.

### BRIEF DESCRIPTION OF THE DRAWINGS

Figure 1 is a graph which shows the rate of color generated in the diaphorase competing substrate test by potassium ferricyanide at different concentrations during the time shown.

Figure 2 is a graph which shows the rate of color generated in the diaphorase competing substrate test by benzoquinone at different concentrations during the time shown.

Figure 3 is a graph which shows the color generated in the diaphorase competing substrate test by phenyl-1,4-benzoquinone at different concentrations during the time shown.

Figure 4 is a graph which shows the rate of color generated in the diaphorase competing substrate test by quinhydrone at different concentrations during the time shown.

Figure 5 is a graph which shows the rate of color generated in the diaphorase competing test by lipoic acid at different concentrations during the time shown. Figure 6 is a graph which shows an assay of alcohol in a saliva sample using lipoic acid during the time shown.

Figure 7 is a graph which shows how the reaction with lipoic acid is made irreversible (here stopped) by control of the pH, ie. lowering the pH to 6.3.

Figure 8 is a graph which shows an assay corresponding to that shown in Figure 5 using 2-hydroxymethyl-6-methoxy-1,       4-benzoquinone (HMMBQ), rather than lipoic acid.

Figure 9 is a graph which shows the linear color inhibition caused by HMMBQ on the color generated by MTT chromogen.

Figure 10 is a graph which shows the color generated in the presence of 18mM of benzoquinone and 1mM of MTT.

Figure 11 is a graph which shows the concentration of alcohol in saliva using benzoquinone as the substrate and the threshold concentration of alcohol of 0.1%.

Figure 12 is a graph which shows the color development due to the chromogen, the presence of a competing substrate, benzoquinone as NADH is being oxidized.

Figure 13 is a graph which shows the linear relationship of color between the substrate concentration and the competing substrate.

Figure 14 is a diagramatic representation of a kit embodying the invention.

3. BRIEF DESCRIPTION OF THE PRIOR ART

The use of dehydrogenase enzymes as specific probes for biological molecules is well known in the art. In general, a biological molecule to be assayed is oxidized by a substrate-specific dehydrogenase, and the resulting product, NAD(P)H, is either assayed directly or converted into a color signal and assayed. When NAD(P)H is assayed directly, the known fact that NAD(P)H has a substantial difference in absorbance at 340 nm than does NAD(P), is used as a measure of the amount of dehydrogenase substrate oxidized. When the NAD(P)H is converted into a color signal, an enzyme or catalyst is used to transfer the electrons from the NAD(P)H to a chromogenic molecule which accepts these electrons with a resulting change in visible color.

A serious problem that existed in all of the art prior to the earlier application is that one equivalent of dye molecule is produced for every biological molecule that is oxidized. This limitation prevented the assay of "high" concentrations of these biological molecules, as the amount of color that would be generated by the complete or near complete oxidation of such a "high" concentration, in accordance with conventional methods, would yield colored solutions that had too high an absorbance to be read without dilution. Additionally, the "high" concentration of dye would lead to solutions wherein dye-dye interaction would cause serious deviation from the ideal as predicted by Beer's law, resulting in solutions that do not have linear relationship of absorbance versus concentration of dye. These difficulties are of more than passing concern. These "high" concentrations (too high to measure), include the normal concentration as well as the super normal concentration indicative of a disease state of most, if not all, medically important biological metabolites. Therefore, when complete or near complete oxidation of a biological molecule was used in the assay methodology common to all previous assays, a massive dilution of the medical sample was necessary to lower the concentration of the biological molecule.

This invention overcomes or mitigates these problems. In the invention described in this specification less than one molecule of dye is created per molecule of NAD(P)H. This ratio of less than one to one is accomplished in a system that contains a diaphorase enzyme that catalyses the NAD(P)H dependent reduction of a chromogen to cause a visible color change, a first substrate for the diaphorase, which causes color to be changed when the chromogen is reduced by NAD(P)H; and a competing substrate for the diaphorase which is an electron-acceptor but which does not undergo a colorimetric change in the same region of the visible spectrum as does the chromogenic substrate. The patent application discloses methods for selecting and identifying competing substrates, all such competing substrates being substrates for the diaphorase enzyme.

This invention also controls the amount of color generated in a colorimetric assay system without the use of diaphorase in a method utilizing the direct NAD(P)H reduction of a chromogen in the presence of a competing substrate for the NAD(P)H.

Diaphorase and lipoyl dehydrogenase have been reviewed (U. Schmidt, P. Graffen, K. Altland & H.W. Goedde, Advances in Enzymology, 32 423-469 (1969) and C.H. Williams, The Enzymes, 13 106-219 (1976).

These publications are noted for (but not only for) their disclosure of lipoic acid derivatives or compounds suitable for use in the invention and for the diaphorases.

The use of diaphorase to produce changes in visible color is widely discussed in the literature. (R.S. Boethling and T.L. Weaver, Clin. Chem ., 25 2040-2042 (1979); (N.J. Hella and S. Weinhouse, Anal. Biochem. , 13 , 322-325 (1965); (C.C. Allain, et al. , Clin. Chem. , 19 , 223-227 (1973); and (F.J. Gella, et al. , Clin. Chem. , 27 , 1686-1689 (1981).

The use of diaphorase in colorimetric assays has also been discussed in the patent literature. U.S. Patent 4,556,634 describes the use of formic acid lower alkyl esters to stop a reaction containing dehydrogenase, diaphorase, NAD(P), and tetrazolium salt. U.S. Patent 4,427,771 discloses an assay method for amylase activity and a method of producing maltose dehydrogenase for use therein. U.S. Patent 4,351,899 describes the use of a test surface containing the dried residue resulting from the impregnation of the surface with a tetrazolium salt, NAD, a dehydrogenase, and an electron carrier. In this patent, diaphorase is not used. U.S. Patent 4,254,222 discusses the assay of lactic acid and beta-hydroxy butyrate via the dehydrogenases lactic dehydrogenase and beta-hydroxybutyrate dehydrogenase.

U.S. Patent 4,271,265 describes the use of diaphorase or electron transfer agents, tetrazolium salts, and NADP in the assay of glutamate-oxalacetate transaminase and glutamatepyruvate transaminase. U.S. Patent 4,247,633 describes the production of a dried, all-in-one reagent for the assay of creatine phosphokinase. U. S. Patent 4,223,090 describes reagents for the enzymatic determination of triglycerides. U.S. Patent 4,215,197 describes the test means and method for creatinine determination. U.S. Patent 4,142,938 describes a method for the determination of triglycerides and glycerol. U.S. Patent 4,024,021 describes a method for the determination of glutamate and glutamic transaminases in biological fluids. U.S. Patents 3,867,259 and 3,867,258 describe the production of lactate dehydrogenase test material. U.S. Patent 3,791,931 also discusses a reagent and method for the determination of lactate dehydrogenase.

In none of the above patents, or in the scientific literature, is there discussed a method of controlling the amount of color that is generated by diaphorase and NADH or NAD(P)H. Indeed, in all of the above patents, one equivalent of dye is produced for every equivalent of NAD(P)H that was present in or produced in the environment as by the oxidation of the substrate. This one-to-one ratio between NAD(P)H and dye produced, imposes serious disadvantageous constraints upon all of the above, previously disclosed methods.

The nature of the problem confronting those skilled in the art may be presented as follows. The vast majority of dyes have millimolar extinction coefficients between 5 and 25 O.D. units. This large absorbance makes it necessary to dilute many medical samples before assay. For example blood and saliva alcohol concentration can range up to 75 mM while the legal value of 0.1% is 22mM. Beta hydroxybutyrate, the major constitute of blood ketones, can be observed in concentrations up to 20 mM, and blood cholesterol concentrations vary from 2 to 10mM. At a 10 mM concentration of test material, dye with a mM extinction coefficient (E-mM) of 5 will produce a solution with an absorbence of 50 O.D. units per cm; a dye with a E-mM of 20 will result in a solution with an absorbence of 200 units per cm. The eye cannot distinguish color difference above 1.5 absorbence units and even the best spectrophotometers cannot differentiate color differences when the solution has an absorbence greater than 3 units.

In the current art, when dehydrogenase/diaphorase assays are used, the sample is diluted so that the concentration of the test material will produce a color within the readable range. This dilution can be either done by hand, or it can be automated as it is currently done in many instruments located in clinical assay laboratories. This requirement for dilution has prevented the production of easy-to-use colorimetric devices for the assay of many biological molecules, such as kits which could be used on location.

Indeed, the scientific and patent literature is surprisingly devoid of attempts at reducing the color that is generated in the assay of biological molecules. However, the problem referred to above has not gone totally unnoticed. U.S. Patent 4,490,465 is a patent that is concerned with reducing the amount of color that is generated in the measurement of biological molecules. This patent discloses a method for reducing the one-to-one ratio of NADH to dehydrogenase substrate that is seen in NAD(P) dehydrogenase reactions. The method disclosed in this patent does not (as does the present invention) reduce the one-to-one ratio of dye produced to NAD(P)H produced from NAD-(P).

The method disclosed in this earlier patent is considerably different from, and has serious disadvantages in comparison with, the method of the invention discussed herein. The previous patent contacts a molecule to be assayed, (A), with two different enzymes that will react with this molecule. One of these enzymes is a dehydrogenase and the other is an oxidase:

A + NAD --- (dehydrogenase) --- NADH + Aox

A + X ----- (oxidase) --------- Aox + Xred

wherein X equals flavin, oxygen, non-NAD electron acceptor. Therefore, at any given concentration of (A) less than one equivalent of NADH is produced. This NADH can be converted into a color by standard methods, for example by the use of diaphorase and tetrazolium salts as has been previously discussed above, to result in the generation of less than one equivalent of dye per mole of (A), the dehydrogenase substrate. It is a key, aspect of that patent as the patentee notes, that the same - the single - substrate is converted by the several enzymes into different products.

The disadvantages of this two enzyme approach are the following: (i) a straight line is not obtained when color is plotted against concentration of (A) (as shown by the patentees); and as the assay device ages on the shelf or in shipping the ratio of dehydrogenase to oxidase enzyme activity will vary due to differing rates of denaturation of these two enzymes. It is the relative activities of these two enzymes that determine the ratio of NAD(P)H produced to substrate consumed. Therefore, the amount of color that is generated at a given concentration of (A) will change with shelf life. This disadvantage renders it impossible to utilize this known technology to produce a device that will reproducibly generate a set color at a set concentration of substrate to be assayed. This serious disadvantage is an unsolvable problem with this known approach.

## OBJECTS OF PREFERRED EMBODIMENTS OF THE INVENTION

There are numerous objects of the invention. One object is to provide an accurate, precise, fast and reliable device which permits quantitative analysis of selected organic molecules normally present in organic liquids or fluids.

Another object is to provide a digital color signal giving an "off"-"on" signal for sought concentration of the unknown.

Another object is to provide an analog to digital chemical color signal device.

Another object is to provide a disposable kit which will give a distinctive color at a threshold concentration of the organic molecule concentration which is to be determined.

Other objects will become apparent to one of average skill in the art in the further description of the invention.

Another object is to provide a system, a method and a device which has numerous practical applications and will contribute to the advancement of technology in the field to which the invention applies.

Another object of the invention is to provide a system, a method and a device for determination of NAD(P)H, which may be generated in situ in the presence of appropriate reactants. Another object of the invention is to provide a system, a method and a device for determination of an organic molecule which will ultimately generate NAD(P)H in the presence of the appropriate reactants.

## DETAILED DESCRIPTION OF THE INVENTION

The invention is useful in the colorimetric determination of NAD(P) or NAD(P)H, a dehydrogenase substrate, or a dehydrogenase enzyme. All of these molecules bear a set relationship to the concentration of NAD(P)H, as will be further disclosed below. Solely for ease in reference in the following section, the discussion will refer to the concentration of NAD(P)H but the usefulness of this invention applies equally well to the detection and assay of all of these molecules.

It has been found in accordance with the invention that the color produced by the diaphorase from the reduction of the chromogen is markedly reduced in the presence of a second substrate for the diaphorase. In accordance with the invention, one of these substrates is a chromogen, and the other substrate is a molecule that irreversibly accepts electrons from the NAD(P)H (the "competing substrate") in a diaphorase catalyzed reaction. With this dual substrate system, it was hoped that the amount of color produced at any given concentration would be reduced and would be at a ratio of less than one molecule of dye per molecule of substrate.

In earlier work, potassium ferricyanide ($K_3FeCN_6$) was first considered as the competing substrate and a tetrazolium salt as the chromogen. There was every reason to believe that this combination would work. $K_3FeCN_6$ is a well known substrate for diaphorase, the reduction of this compound was expected to be irreversible. Further, the affinity of these pairs of substrates for the enzyme, as measured by the substrate Km, is generally within the same range. Massey (Biochem. Biophys. Acta. (1960, 37 , 314-322) has disclosed that the Km of $K_3FeCN_6$ is 0.27 mM, while F.J. Gella et al (F.J. Gella, M.T. Olivella, F. Pegueroles, and J. Gener, Clin. Chem. (1981), 27 , 1686-1689) has disclosed that the Km for the tetrazolium salts are: 0.87 mM for INT, 1.25 mM for MTT (thiazolyl blue tetrazolium bromide), and 2 mM for NBT (nitro blue tetrazolium chloride).

Therefore, it was a surprise to find that this system did not work as was expected. In further work, it was found that the $K_3FeCN_6$ was preferentially reduced until it was exhausted, and only then was the tetrazolium salt chromogen reduced to produce color. From this work it was concluded that the two substrates did not share random access to the diaphorase activities. Therefore, in cases where the concentration of $K_3FeCN_6$ was greater than the concentration of NAD(P)H assayed, no color was produced in the system. In cases where the NAD(P)H concentration was greater than the concentration of $K_3FeCN_6$, the final color was that calculated by subtracting the concentration of $K_3FeCN_6$ for that of the NAD(P)H and multiplying the resulting number by the known extinction coefficient of the reduced tetrazolium chromogen.

In further work, the use of 1,4-benzoquinone as a competing substrate was considered. Benzoquinone has never before been implicated as a substrate for diaphorase, so this choice may have seemed illogical, but it was reasoned that this molecule was antiaromatic, and easily reduced. Therefore, it seemed a reasonable candidate substrate. It was found that benzoquinone is indeed a substrate for diaphorase. However, benzoquinone behaved like $K_3FeCN_6$ and gave identical results. That is, no color was generated by the system until the benzoquinone was exhausted.

In additional work, the definitional requirements for an ideal second or competing substrate for the purpose of a main embodiment of the invention were discovered. A second substrate is in accordance with these findings, a compound which meets a three-way test.

The first requirement for the substrate is that it be or caused to be an irreversible substrate for the diaphorase. In accordance with the invention, the reduction of the competing substrate should be irreversible. This property can be determined as follows.

Diaphorase is mixed with a concentration of NAD(P)H capable of producing a dark color, for example 2 mM; a concentration of tetrazolium salt chromogen that is greater or equal to the concentration of NAD(P)H, for example 4mM, and a suitable concentration of test candidate substance, for example, from 1 to 500 mM. These reagents are allowed to react to completion, and the end point color is determined. When the second substrate is an irreversible substrate for diaphorase, the color that is generated at end point in the presence of that competing substance, will be less than that generated in a control tube wherein the competing substance is omitted. The second requirement for a suitable second substrate to be used in accordance with the invention is that the color generated by the chromogen must be at a˙ non-zero initial rate when the second substrate is present in sufficient quantities to inhibit the diaphorase. This property is determined as follows: diaphorase was mixed with a quantity of NAD, for example 0.1 to 25 mM; an excess of dehydrogenase enzyme, for example alcohol dehydrogenase; a large excess of concentration of a substrate for the dehydrogenase enzyme, for example alcohol at a concentration of between 10 and 1,000 mM; a tetrazolium salt chromogen, for example MTT at 2 mM; and the second or competing substrate at a concentration of between 4 and 500 mM. The entire reaction mixture is placed in a spectrophotometer, and the kinetics of color production measured. For a first embodiment, a suitable second substrate is one which causes a color generation at a non-zero rate, that is less than the rate that is seen in the absence of the competing substrate. Suitable second substrates are described further below.

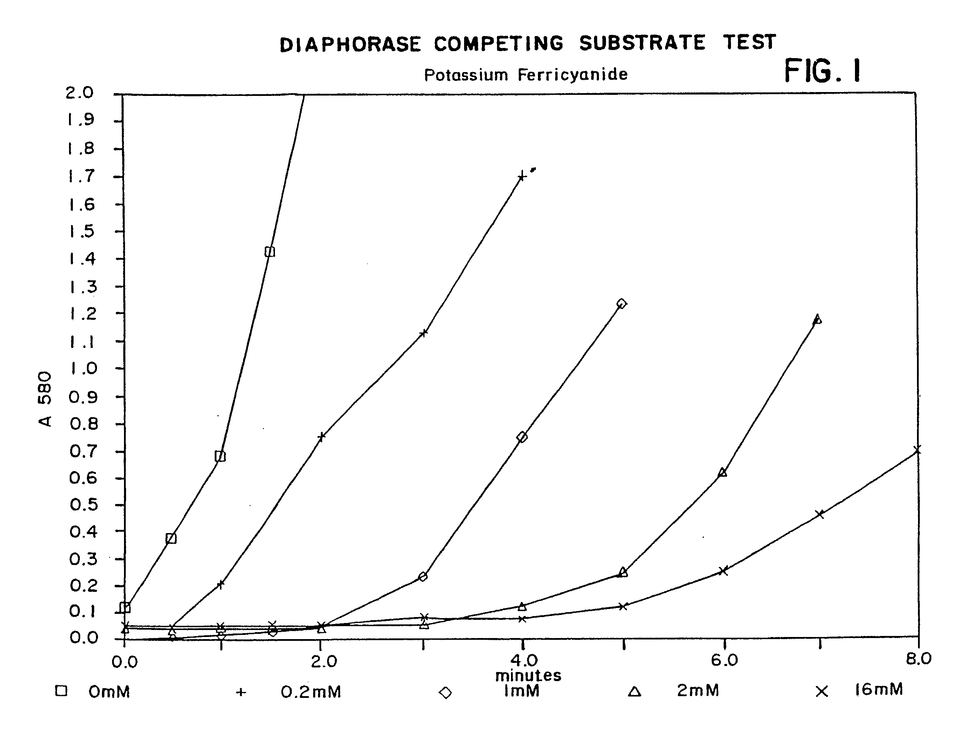

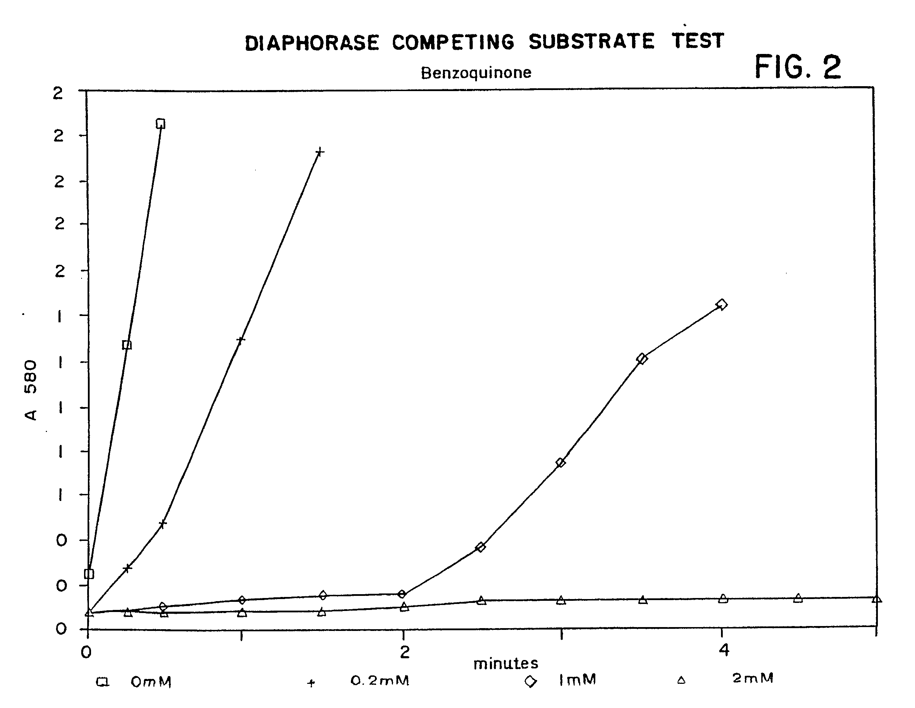

Where a rate of zero color production was observed until the competing substrate was exhausted, and only then color produced, the competing substrate does not qualify for the purpose of this embodiment of the invention. Such was the result for both $K_3FeCN_6$ and 1,4-benzoquinone as disclosed in Figures 1 and 2 presented and discussed herein.

The third requirement for a suitable substrate candidate is that the amount of color that is generated at each of several concentrations of NAD(P)-H is linearly related to that concentration. In this third test, diaphorase is mixed with several different concentrations of NAD(P)H, for example INT at 2mM; and a concentration of competing substance that is greater than the highest concentration of NAD(P)H utilized, and is constant throughout the test.

These reactions were set up in several different test tubes, each tube containing a different concentration of NAD(P)H and the reaction allowed to go to completion. The color that is generated at each NAD(P)H concentration is measured and plotted against the concentration of NAD(P)H that was in that sample. Competing substances - suitable substrates - will only pass this test if this test yields a straight line over at least a section of the NAD(P)-H concentrations.

There are therefore in accordance with the invention, two main embodiments: a first wherein the affinity of the enzyme (the diaphorase) for the two substrates is within approximately or within the same range of magnitude; a second embodiment. wherein the diaphorase has a greater affinity for the completing substrate than for the other substrate. In the first embodiment, the color generated by the chromogen is at a non-zero initial rate; in the second, the rate of color production from the chromogen is nil until the competing substrate is totally (or virtually) oxidized.

The relative mole percent of chromogen to competing substrate useable in accordance with the invention, varies over a wide range. One skilled in the art can determine readily the optimum relative proportions of the two substrates for the diaphorase. It is evident that the relative amounts of the two substrates will vary depending on the intensity of the colored dye generated by the chromogen in the absence of the diaphorase. If the chromogenic substrate is such that it would not generate a very large excess of dye beyond that which can be readily colorimetrically measured, the proportion of competing substrate that needs to be present is in a smaller proportion than if the chromogen is such as to generate a larger or intenser excess of color determinable colorimetrically. Likewise the relative proportions will depend on the nature of the substrate and the amount of the substrate in solution which is sought to be determined. In general, the proportion of competing substrate is at least that much as will cause a decrease in the amount of colored dye generated by the diaphorase reaction on the chromogen. The maximum of competing substrate should not be such as to cause the amount of colored dye generated to be so small as not to be measured colorimetrically. The relative molar proportion of competing substrate to chromogen and vice versa is also dependent on the amount of NAD(P)H present in the reaction. The amount of NAD(P)H present should be enough to cause the reduction of both the chromogen and the competing substrate. In general, the relative molar proportion of competing substrate to chromogen ideally should be equal to or greater than 1 but either one of the substrates may vary and be in excess of the other under certain circumstances, ranging to a ratio greater than 400 to 1 with respect to the other, and depending on the amount of color reduction required. In general, though an excess of chromogen over competing substrate is not desirable for this could generate additional colored dye after the competing substrate is exhausted. The reverse condition will advantageously extend the colorimetrical range of color response.

Of course what has been said above applies likewise for the aspect of the invention where rather than developing a colorimetrically measurable color there is decreased the amount of color from an intense to a color within a colorimetrically determinable range.

In the aspect in the embodiment of the invention where the competing substrate is irreversibly reduced prior to the chromogen reduction, the relative molar proportion of the two substrates are in the same proportions as explained above. It is important however, that if one wishes to generate color, that there be enough NAD(P)H present to provide for the catalytic oxidization of the competing substrate first to be completed and then allow for the reduction of the chromogen to generate the color.

It has been discovered in accordance with the invention, that lipoic acid and derivatives (as defined hereafter) are suitable competing substrates. This, however, came surprisingly after a number of failures. Initially this concept was rejected for several reasons. In order to pass test one, as described above, the reaction between NAD(P)H and the competing substrate must be functionally irreversible. This is because the reaction between the NAD(P)H and the competing substrate must be functionally irreversible. Therefore, if a reversible reaction with the competing substrate were to occur, this reaction would freely reverse, and the regenerated NAD(P)H resulting from this reaction will again react and partition itself between the competing substrate and the chromogen. This process of reaction and partitioning will continue until either the available pool of chromogen is exhausted, or until all of the NAD(P)H is used up to produce dye to cause one equivalent of dye per equivalent of NAD(P)H initially present.

It is well known that the reaction between NAD(P)H and lipoic acid or the derivatives described below (hereafter called lipoic acid compounds) is freely reversible in nature. Indeed, the reaction that occurs in the course of cellular life is the reverse of the desired reaction. Lipoic acid is normally utilized in the production of NAD(P)H, not as an irreversible trap for this molecule. The reversibility of the lipoic acid/NAD(P)H reaction has been well documented in the literature and the equilibrium constant for this reaction measured. This equilibrium constant again argued against the success of using lipoic acid (or the derivatives). Indeed, there is at least one report of an assay for the production of NADH from NAD and reduced lipoic acid amide, DL dihydrolipoamide (D.J. McKay, and K.J. Stevenson, Biochemistry 18, 4702-4707 (1979)), clearly indicating the reversible nature of this reaction.

When 75 mM lipoic acid was used in the above test system as the soluble, tetramethylammonium salt, the solutions failed the first test outlined above. Surprisingly, when a supersaturated solution of lipoic acid was tested, it passed all three tests. This favorable result was obtained when lipoic acid was incorporated as either the free acid or salt or as a derivative thereof.

No clear explanation is available as of now for this completely surprising and unprecedented result. With hindsight, it was hypothesized that reduced lipoic acid is less soluble than is oxidized lipoic acid. In a super-saturated solution, the reduced lipoic acid is removed from the solution by precipitation, whereby it is not available to react with the enzyme in the reverse reaction. Other explanations are possible and may be a cause of this success in this invention

These results were also obtained when a solubilizing wetting agent was used in the above experiment. After obtaining this unexpected result the addition of other ingredients to this reaction mixture was considered to determine whether and how the lipoic acid reaction (or that of the competing substrate generally) could be made to be irreversible.

The use of reagents to make the lipoic acid/NAD(P)H reaction (or the reaction of the equivalent reactants) irreversible has not been discussed in either the patent or the scientific literature so that these studies too were carried out without any suggestion from the prior art.

In this manner, it was discovered that the presence of zinc ions would allow even a saturated, in contrast to supersaturated, solution of lipoic acid (or its equivalents) to pass the above tests. Other reagents that enable lipoic acid to pass these rigorous screening tests are iodoacetic acid, and oxidized 2-mercaptoethanol, for instance produced by bubbling oxygen gas through a solution of 2-mercaptoethanol.

From these successes, it was reasoned that in accordance with the invention the added reagents must either: (i) chelate (bind to) reduced lipoic acid (herein called dehydrolipoic acid which is intended to include all of the reduced lipoic acid derivatives discussed herein) with a greater affinity than the chelate oxidized lipoic acid; or (ii) react with dihydrolipoic acid, but not, or to a lesser extent, react with lipoic acid.

Several reagents selected using these criteria have been successfully tested, including: metal ions like ferric ion, mercury ion, chromium ion, chloroacetone, dichloroacetone, methyl iodide, and all disulfide compounds currently attempted. All compounds, in addition to those specifically disclosed that will allow lipoic acid in solution to pass the above three tests, are specifically operative and intended to be covered by this invention. In addition, all compounds other than lipoic acid, used either singly or in conjunction with other compounds, that in the presence of a suitable chromogen, pass the three tests disclosed above are intended to be covered by the invention and their absence from being explicitly named from this specification in no way is intended to exclude these compounds or combinations of compounds.

In accordance with the invention, there is used a mixture of a competing substrate and a prodye or chromogen, often a tetrazolium salt to reduce and control the amount of color that is generated in the presence of NAD(P)H. In most cases, the NAD(P)H is produced by the action of a specific dehydrogenase on its substrate. It was found, in this invention, that the amount of color generated plotted against substrate concentration gives a straight and reproducible line. Thus the concentration of an unknown amount of substrate is determined by comparison of color formed by the unknown sample and a linear standard curve produced by known samples. This color control system can be present in aqueous solution or suspension, or it can be incorporated into a diagnostic device in a dry format, particularly a dry film format as will be further discussed herein. In addition, the diagnostic device comprises a dry film, a dehydrogenase and if necessary, a trap for the oxidized substrate, customary necessary buffer salts, and NAD(P)H.

One skilled in the art is quite capable to identify additional substances which are suitable second substrate since the parameter or tests for such substrate are disclosed herein.

The lipoic acid compounds which can be used in the invention as the competing substrate are those which are reduced in the presence of diaphorase and NAD(P)H and include for example the following: the esters, such as alkyl, e.g. lower alkyl esters (from 1 to 6 carbon atoms), aryl esters, (including alkaryl esters), e.g. having 6-10 carbon atoms including benzyl and phenyl esters, amides like dihydrolipoamide or lipoic acid wherein the carboxy group has been replaced by a sulfonamide group. Also useful derivatives of lipoic acid are those which have substituents on the carbon atoms of the heterocyclic ring and/or on the-$(CH_2)_4$-COOH chain, including the lengthening of the alkylene group to 6 or more carbon atoms, (or shortening it) or having another atom instead of the sulfur atoms in the ring. Illustrative substituents include keto, hydroxyl, alkyl (e.g. lower alkyl), fluoro, etc. Illustrative compounds are the following: the amide of 4-oxalipoic acid, 4-methyllipoic acid, 5-ketolipoic acid, lipoyl pyridoxamine, 2-fluorolipoic acid, 7,7-difluorolipoic acid, 8 methyl-7-fluorolipoic acid and 8-methyl-7,7-difluorolipoic acid. Other lipoic acid derivatives are known and such are disclosed in Biochemistry and Chemistry of Lipoic Acids, Schmidt et al in Advances in Enzymology, 32 , 423-469 (1969), which is incorporated herein by reference. Specifically included are those derivatives in which the lipoic acid is bonded to amino acid through an amide bond. The synthesis of many substituted lipoic acid analogs which are suitable for this invention, are disclosed in the 1973 Ph.D. Thesis by Hanan N. Alkaysi, University of Kansas, and available from University Microfilms International, Ann Arbor, Michigan.

In accordance with the invention, the competing substrate may be reduced completely before a visible color will be generated from the chromogen. This aspect of the invention contrasts with that described above where the color is generated gradually from the chromogen as the second substrate is reduced. In this aspect herein called "threshold gating control", when the amount of color generated by the chromogen is plotted according to the second test described herein all color found is plotted against the NAD(P)H present or found in the reaction, it shows a generated linear relationship after a delay which corresponds to the time or amount necessary for the competing substrate to be reduced, during which delay no visible color is generated. During that period, essentially no chromogen is reduced, at least not enough to generate a visible color change. In that embodiment of the invention, the affinity of the enzyme for the substrate is greater for the competing substrate so that no reduction of the chromogen takes place until all, or virtually all, of the competing substrate is exhausted. Under certain circumstances, the color development will start even though there may still be an amount of about 2mM of competing substrate present.

Numerous other diaphorase competing substrates in addition to those described above are known as disclosed, for instance, in Chromogenic Substrates and Dyes and Dye Intermediates in Sigma Chemical Company's catalogue. As described above, for one embodiment of the invention, the competing substrate is reduced currently (at least for a portion of the time, preferable for the major portion of the time), while the chromogen is being reduced and generates color. In this embodiment of the invention, the competing substrate may be considered as providing "random access" with the chromogen to the diaphorase. For the second major embodiment of the invention, the competing substrate is a preferred substrate for the diaphorase with respect to the chromogen. Unlike the first embodiment, no color will be generated unless a predetermined amount - the threshold amount - of the substance to be measured is present. In this situation color is only generated when the NAD(P)H is present in an excess over the amount necessary for the reduction of the competing substrate. This amount is predetermined by determining at what minimum concentration of NAD(P)H a color will be developed from the substrate selected. Thus, this system allows a digital "on-off" reading which determines whether NAD(P)H is present in a concentration greater than the predetermined threshold. The system for determination of the amount of substrate to be tested contains varying concentrations of NAD(P)H up to and in an amount in excess of the minimum amount.

For the first embodiment of the invention, various competing substrates in addition to the lipoic acid compounds described above, can be used. Typical are various benzoquinones, especially the lower alkoxy (e.g. 6-substituted benzoquinones and/or benzoquinones hydroxy lower alkyl (e.g.

methyl) substituted. Benzoquinones, especially the 1,4-benzoquinones forms a suitable class.

Such benzoquinones are known, see for instance CRC Handbook of Chemistry and Physics 1981-1982 Edition. Such benzoquinones are useful if they pass the three tests described above.

Another suitable class of preferred compounds well suited as competing substrates are substituted pyridones such as 4-pyridone-N-acetic acid salts diiodo-substituted. In general, it has been found that unsaturated molecules that are easily reduced and are anti-aromatic are suitable competing substrates; also certain dithio compounds especially those that contain hydrophobic regions in this molecule are suitable competing substrates. For example, dibenzyl disulfide, dithiodibenzenes, dithiodinitrobenzoic acid and tert -butyldisulfide.

Likewise these compounds are known and are listed in the above referred to Handbook. These compounds too are in accordance with the invention, called to pass the three tests described above.

For the other embodiment of the invention, the "threshold gating control test", various inorganic salts like the alkaline earth metals and alkaline metals of ferricyanides are useful. Also useful are benzoquinones, especially unsubstituted or with aromatic substitution like phenyl and other aromatic compounds like benzoquinhydrone (quinhydrone). Other aromatic compounds which in the presence of a chromogen will react with NAD(P)H preferentially are suitable including those having a single or multiple aromatic rings (fused or not) which may have various substituents. These compounds may generate a color when irreversibly reduced in the presence of NAD(P)H. This color generation does not completely overlap the region for chromogen color generation.

The rings of these compounds may be hydrocarbon or heterocyclic 5 or 6 membered rings (with one or more heteroatoms) like pyridines, thiophenes, furans or pyrroles; they may also be condensed or polycyclic derivatives like indoles, benzofurans, benzothiophenes, quinolines (isoquinolines), carbazoles, acridines, imidazoles, thiazoles, pyrazines, pyrimidines, purines or pteridines, generally substituted on the ring. The only requirement for these heterocyclic ring compounds is that they be substrates for the diaphorase. In general, these ring systems which are anti-aromatic are likely to be substrates. The presence of a hetero atom/hetero atom or hetero/carbon bond is not necessary; for example, 7,7,8,8-tetracyanoquinodimethane is a suitable substrate. Other compounds will be apparent to one skilled in the art.

The second embodiment of the invention provides a convenient and rapid test for determining a predetermined concentration of a substrate desired to be measured. The device of the invention can be precalibrated so that if or when the concentration of the substrate reaches the predetermined concentration, the color will be generated; if the substrate present is less than that concentration (or absent), no color will be generated. Such "positive" or "negative" test can be conveniently used, for instance, to determine the concentration of alcohol in saliva, or in blood, or sugar in blood or serum. For instance, when the device is calibrated for 0.12 alcohol the color will develop if the concentration in the sample (e.g. saliva) is 0.12 or higher. It is evident that such device is convenient for a sobriety test.

In accordance with the other embodiment of the invention, the second or competing substrate is a substance which is irreversibly reduced by NAD-(P)H in a diaphorase catalyzed reaction which decreases by this reaction the amount of color produced in the system where the competing substrate would not be present in the system from a chromogen in the diaphorase catalyzed reactions. The amount of color produced in the presence of the second substrate is in a ratio less than one molecule of dye per mole of NAD(P)H assayed.

The system of the invention can be a liquid system or in a dry format. In the latter, it may be a diagnostic kit. Such diagnostic kit or device comprises a film which can comprise a multilayer sandwich with one layer containing the color control system, and another layer containing the dehydrogenase and a trap for the reacted substrate. The film or film sandwich is suitable for incorporating into a round or rectangular capillary of controlled volume. In this embodiment, the test fluid is drawn to the controlled volume capillary by capillary action, whereupon, it is acted upon by the dehydrogenase to convert all of the specific test material into NAD(P)H and oxidized substrate. The oxidized substrate can, if desired, be trapped in this layer to ensure that the reaction goes to completion and to remove any inhibitory effects of the oxidized substrate.

The NAD(P)H diffuses to the diaphorase which is located in the same film layer or in another layer of the sandwich. The diaphorase uses the NAD(P)H to reduce the competing substrate, for instance the oxidized lipoic acid (or a lipoic acid derivative) and to reduce a tetrazolium salt or other suitable compound as described herein.

The amount of color generated is dependent upon the preset ratio of chromogen to competing substrate. Thus the amount of color that is generated in the capillary is representative of the amount of test material that was contained in the test sample, and the amount of color generated is within the range of color that can easily be detectable by eye.

In accordance with the invention, the system (and the process) of the invention can be used to assay NADH or NAD(P)H directly. In another embodiment, the invention is useful to assay and determine the amount of NADH or NAD(P)H generated by any chemical (also enzymatic) or electrochemical method. The source of the NAD(P)H is not important to or a limiting aspect of this invention. The scope of this invention extends far beyond the specific examples used for purpose of discussion and illustration in the experimental methods section.

There is also, in accordance with the invention, a third embodiment whereby it is possible to reduce the color generated upon the direct NAD(P)H dependent reduction of a chromogenic molecule to within a readable range, without the use of diaphorase.

It was found in accordance with the invention, that in the system - and the method - the diaphorase can be omitted. Instead, a non-protein catalyst is used. This is greatly advantageous, as the cost of diaphorase can be appreciable in total product material cost, and the diaphorase is more susceptible to certain temperature, pH (and other conditions which are not optimum for that enzyme) than a catalyst that is an organic or inorganic molecule (but not enzymatic like diaphorase).

In accordance with this embodiment of the invention, there is used an electron-carrier catalyst which oxidizes NAD(P)H and transfers electrons to the chromogen forming a formazan in the presence of the reactant. The catalyst, as explained further below is an organic or inorganic molecule other than diaphorase.

Such electron-carrier catalysts are known in the art. However, in the methods of the prior art (for instance, U.S. patents 4,024,021; 4,351,899 and others) one molecule of dye is produced per molecule of NAD(P)H. In the present system as described below, there is produced less than 1 molecule of reduced chromogen per 1 equivalent of NAD(P)H present or produced in the system of the invention.

The third colorimetric assay system of the invention comprises a prodye or chromogen which is an electron-acceptor for NAD(P)H and which is capable of changing color in the visible range. A typical chromogen is a tetrazolium salt. In the system of the invention, the chromogen may be colorless to start with and develop a color upon reduction or the compound may be colored to start with and become colorless as the reaction is completed, or the chromogen may be a light color and change to a deep or other distinctive color, like from yellow to deep blue or purple.

The system of the invention also includes NAD(P)H which may be generated in situ as described hereafter, and a reactant (or reductant) which is capable of accepting electrons from reduced chromogen and/or from NAD(P)H and an electron-carrier catalyst which is capable of transferring electrons from NAD(P)H to the chromogen and at least theoretically, vice-versa.

The reactant used in the invention is capable of preventing the accumulation of reduced chromogen and thus the development of color attributable to the reduced chromogen. Thus, if the chromogen is a light yellow, for instance and the reduced chromogen deep blue, the reactant reacts with the reduced chromogen as it is formed, causing it to revert virtually instantaneously to or essentially causing it to remain in its chromogen state (yellow) and not to develop the deep blue color. Thus, the reactant, in accordance with the invention is capable of regenerating the chromogen. These conditions prevail until the reactant is exhausted.

Thereafter, and in the presence of NAD(P)H, the full color of the reduced chromogen will develop virtually instantaneously, in this illustration, the deep blue color. Because of the nature of the reaction, the presence of essentially a trace amount of chromogen will generate the color, for practical purposes approximately 1 mM. The concentration of reactant with respect to the chromogen is not critical and may for instance be 50, 100, 200, 300 mM, or more. When there still is NAD(P)H present and the reactant is depleted, i.e. an excess of NAD(P)H is present in the system over the amount of electron-accepting ability of reactant, color will instantaneously develop. For purposes of definition, this is the "threshold" amount of NAD(P)H. Thus the system gives a digital reading of two states, and two states only: an "off" and "on" state, the first corresponding to no "color", the second to "color". By "on" and "off", there is meant as described above a "change of color" not only "color" to "colorless" or vice-versa. Thus, unlike the first embodiment of this invention (and unlike many conventional prior art methods and systems), in the present invention the concentration of the NAD(P)H does not correspond to an intensity of color generated, nor it is necessary to perform any plotting (color generated vs. standard color curve) to know the concentration of the NAD(P)H or of the organic compound (or substrate) which generated the NAD(P)H, when such substrate is present.

In accordance with the teaching of the third embodiment, it will be seen that in cases where the color is in an "on" state there may be and there is generally likely to be present an excess of NAD(P)H in excess of the trace, or as stated above about 1 mM chromogen necessary to develop the color upon depletion of the reactant. As an illustration, in a system which at starting conditions contains 20 mM of reactant, 1 mM of chromogen and 40 mM of NAD(P)H there results (in the situation where 1 mole of reactant accepts 2 electrons from 1 mole of NAD(P)H), 20 mM of depleted reactant, 1 mM of reduced chromogen and 19 mM of excess NAD(P)H. This excess of NAD(P)H does not convey relevant information for the purpose of the invention.

The system will have given an "on" reading at the instant when and by the development of strong color when a trace or in this instance approximately 1 mM of reduced chromogen will have been formed by reaction with the 1 mM of NAD(P)H.

Also in accordance with the third embodiment, it will be appreciated that the invention is not limited to absolute numbers, as illustrated above for instance for the 1 mM. What is more significant is the color formation attributable to the reduced chromogen or dye. Ideally the extinction coefficient of the reduced chromogen is so high that less than 1 mM of reduced chromogen is required to cause an abrupt and strong positive color development or signal. The extinction coefficient of reduced chromogens are known and when not known or readily available, can be readily determined by one of average skill in the art without undue experimentation. Ideally, therefore, a chromogen is to be selected which in the reduced state gives a "clear", sharp, unequivocal, distinct color chance in the system of the invention upon reaction of the NAD(P)H.

In the situation where reactant is not depleted when NAD(P)H is depleted, it is evident that chromogen will still be present but no excess reduced chromogen be formed and hence no color change occurs.

There is an important aspect of the third embodiment which will become more apparent from the discussion which follows.

In accordance with the invention, it has been discovered that the concentration of NAD(P)H can be measured very accurately and efficiently in an "on-off" digital manner. Where the reactant is reduced until essentially depleted, a visible color develops if and only if excess NAD(P)H is present; if none is present, no color develops. Thus, in accordance with the invention, a particular pre-selected concentration of reactant corresponds to a threshold accumulation of NAD(P)H. If it is above the threshold, a color will develop when the reactant is exhausted, but no color, when the reactant is not exhausted and the NAD(P)H is below the threshold. In accordance with the invention, several concentrations of the reactant are pre-selected and the development of color corresponds to concentration of the NAD(P)H in the system to be determined, whether it is a liquid or preferably, a device with a solid support for the reactants.

The determined concentration of NAD(P)H also corresponds to the concentration of the organic compound which is sought to be determined, if one is present and which generates NAD(P)H (in the presence of the appropriate dehydrogenase).

At the threshold, the concentration of reactant is equal to 1 or 2 times that of NAD(P)H, this being dependent on whether the reactant is capable of accepting 2 or 1 electron from the NAD(P)H. Where the reactant is capable of accepting 2 electrons, the relationship is 1 to 1, and it is 2 to 1 when the reactant is capable of accepting I electron from the NAD(P)H.

In addition to the system of the third embodiment, and various devices, the third embodiment provides a method for measuring NAD(P)H which may be generated in situ by an organic substrate the concentration of which is sought to be determined. In that embodiment, an appropriate dehydrogenase specific for the substrate is also present, as will be described further hereinafter.

In accordance with the third embodiment, the ingredients of the system are brought together as a reaction mixture which contains the chromogen, the electron-accepting reactant and the catalyst. To the mixture there is added NAD(P)H until a color change occurs. The color change is indicative of the exhaustion of the reactant and that chromogen has been reduced to a strong visible color.

In accordance with the method, various linearly related concentrations of the reactant can be provided in different regions of an assay device. For example, linearly related differing reactant concentrations can be situated along the "X" axis of a device containing this system. Thus, an "on" signal will be generated at a different linearly related concentration of NAD(P)H or substance that generates NAD(P)H along this "X" axis. When the reactant is situated in this matter, increasing either linearly along the "X" axis or in steps of increasing or decreasing concentration, the amount of NAD-(P)H that is introduced into the system to effect measurement, will be determined by visual inspection of the distance that "on" signal, that is to say, color change, is propagated along the "X" axis.

This embodiment provides a practical measuring device of numerous industrial and commercial applications.

When in accordance with the invention it is desired to measure the concentration of an organic substrate, the method comprises bringing together the chromogen, the electron-accepting reactant, the catalyst, NAD(P)+ and a suitable specific dehydrogenase for the organic substrate to be measured. To this reaction mixture there is added in increasing amounts of the substrate to be measured, causing the substrate to be oxidized, the NAD(P)+ to be reduced to yield NAD(P)H and the reaction to proceed until color is generated, as described above. At that point the reactant is depleted and NAD(P)H generated from the substrate reacts with chromogen to yield reduced chromogen and a highly visible color change indicative of the concentration of the substrate.

Generally the reaction is carried out in a buffered environment at a pH preferably optimum for the catalyst and enzyme used, as is described hereinafter and at optimum temperature.

The reaction may be carried out in a liquid or on a suitable physical carrier which allows for the reactants: to react as described.

Although the invention is not limited by any particular scientific theory or principle, the third embodiment can be considered as having two principal forms.

In the first form, the reactant reacts with the reduced chromogen as it is produced by the catalytic reduction of the chromogen and thus prevents the formation of colored chromogen. When the reactant is all consumed as discussed above, colored (reduced) chromogen is then generated catalytically, where color is indicative of the presence of the organic molecule, the concentration of which is sought to be determined.

In the second form, it appears that the reactant will accept electrons directly from, and thus react directly and preferentially with, the NAD(P)H in the system, or as it is formed from the organic molecule. When it is exhausted, the NAD(P)H will react with the chromogen forming reduced chromogen, as described above.

It is not essential that a distinction between the two forms be made in the practice of the invention since in both cases color produced by the reduced chromogen readable (without dilution) in the visible range will be formed.

It will have become apparent from the discussion of the invention that where the threshold is reached - and an "on" signal is read - there is produced less than 1 equivalent of dye (reduced chromogen) per equivalent of NAD(P)H at any concentration of NAD(P)H, this being another distinction over the prior art.

Reactants which are useful for use in the invention are generally weak organic and inorganic oxidizing agents which are stable in an aqueous environment at the operative temperature and pH. A great variety of reactants are available for use in the invention. A screening test has been developed which allows the determination of suitable candidate reactants without undue experimentation.

For the test an equivalent of NAD(P)H is added to an equivalent of chromogen and a catalytic amount of diaphorase in a flask, and the resulting dye is aliquoted into test tubes. A candidate reactant is added to the test tubes. A reactant passes the test if the dye color is removed, i.e. the reduced chromogen has been reverted to chromogen. For best results it is also necessary that the reactant be incapable of inactivating the diaphorase or be inert with respect to another catalyst used. For this purpose, the following test is used. A known amount of reactant, for example 10 mM, is mixed with about 1 mM chromogen, and a catalytic amount of diaphorase. Aliquots of NAD(P)-H are added to this reaction medium, 2 mM at a time. The reactant is said to pass the test if the solution containing 4 mM demonstrated the presence of reduced chromogen or dye. This second stage test also will determine if the reactant has a 1 to 1 or a 2 to 1 reactivity with the dye.

The second test discussed above also identifies a reactant that will not accept electrons from the reduced chromogen, but instead acted by preferentially receiving electrons from the NAD(P)H. In general; any similar test that tests for the absence of color change when reactant is substantially present can be used for the tests described as will be obvious to anyone with average skill in the art.

In accordance with the invention, by weak oxidizing agents for use in the invention, there is meant any compound or element that is stable and for which is more electron accepting that is NAD-(P)H/NAD(P) as measured by standard electronegativity assays, for example as discussed in the well known textbook "Physical Chemistry" by Walter J. Moore, published by Prentice-Hall, New Jersey (1972), incorporated herein by reference. Preferably the compound should be stable for about an hour in aqueous solution. Examples of molecules that are more electron-accepting than the NAD(P)H/NAD(P) couple are organo-metal salts, for example: ferric salts, e.g. the triethyldiamine chloride salt of Fe(III), the Fe(III) citrate complex, the Fe(III) EDTA complex, and the Fe(III) sorbitol complex; cobalt, for example the Co (III) compounds hexamino cobalt (III) chloride, potassium hexacyanocobaltate (III), and sodium hexanitrocobaltate (III), and the like; and similar salts or other, electronegative transition metal ions. A reactant of choice is ferricyanide, and its alkali metal salts like sodium, potassium, and other equivalent water-soluble iron salts, or other equivalent.

The choice of reactant is not limited to metals, but rather to the requirement that it be an electron-accepting molecule that is stable in aqueous solution. Thus, inorganic oxidizing agents like sodium or potassium periodate have been utilized as reactant molecules, as have organic oxidizing agents, for example dithiols, like aromatic (e.g. benzyl)-nitrosubstituted thiols, typified by para-nitrobenzyldisulfide, 2,2'-dithiolbis-(pyridine-N-oxide), and 2,2'-dithiobis(4-tert-butyl-1-isopropylimidazole). Various organic peroxides are suitable inclusive of hydrogen peroxide, other organic peroxides, for example the peracids of lower alkyl acids, like acetic, formic and the like, and molecules of the general structure R-O-O-R' wherein R and R' are or are not equal and are members of the general class of aliphatic (e.g. lower alkyl), or aromatic, or cyclic hydrocarbons, or are members of the above classes containing substitutions of nitrogen, oxygen, thio, cyano, halide (like bromo- or chloro-) or the like.

It should be especially noted that heterocyclic organic compounds can be utilized as the reactant, like substituted triazones, for example melamine, and the halo substituted melamines like trichloromelamine, and also halo substituted N-trifluoromethylflavin.

Electron-carriers or transfer catalysts suitable for the third embodiment are substances which have oxidizing activity on NAD(P)H to NAD(P) + and no detrimental action on the coenzyme cyclic reaction. Such catalyst include other than diaphorase, polycyclic (generally aromatic) unsaturated dyes of various subclasses. Illustrative are benzoquinones, especially with aromatic substitution like phenyl and other aromatic compounds like benzoquinhydrone (quinhydrone). Other aromatic compounds which in the presence of a chromogen will react preferentially with NAD(P)H are suitable including those having single or multiple aromatic rings (fused or not) which may have various substituents. These compounds may generate a color when reduced in the presence of NAD(P)H. Specific typical useful color-generating electron-carriers include phenazonium methosulfate (PMS), N-methyl-PMS, meldola blue, pyrocyanine, N-methylphenazonium methosulfate, methylene blue, riboflavin alloxazine, 9-amino-1,2,3,4-tetrahydroacridine, substituted anthraquinones (saturated or unsaturated) which are color-generating are various amino-, alkyl- (especially lower alkyl, like methyl), halo-(like chloro-, or bromo-), hydroxy-, nitro-sulfonic acid, alkoxy-(like methoxy) anthraquinones. Such anthraquinones are listed in the Handbook of Chemistry and Physics, 62nd Ed (1981-1982) (Section C) and are known under such common names as purpurin, flavopurpurine, hydroxychrysazin, anthragallol, quinalizarin, tufiopin, hystatazin, aloemodine, alizarin; other aromatic color-generating compounds like azulene, benzaurin, and other equivalent compounds having the same function and effect. When the selected catalyst generates color, the color should not be generated in the same visible spectrum where the color of the chromogen upon reduction will be generated.

Generally the embodiment of the invention involving diaphorase is more efficient; however, where conditions of the system are such that they might tend to adversely affect the stability of the enzyme, the non-protein electron-carrier catalysts are preferred. Benzoquinone is not a desired catalyst electron-transfer compound for the invention.

Suitable chromogens for use in all three embodiments are abundant. Conveniently they are tetrazolium salts. Illustrations are 2-(2'-triazolyl)-3,5-diphenyl tetrazolium bromide (MTT), 3,3' dimethoxy-4,4'-diphenylene)bis[2-(p-nitrophenyl)-5-phenyltetrazolium chloride] (NTB), 2-(p-nitrophenyl)-3-(p-iodophenyl)-5-phenyltetrazolium-chloride (INT) or 2-(4,5-dimethyl-2'-thiazolyl)-3,5-diphenyltetrazolium-bromide (4,5-MTT). The concentration of the tetrazolium salt is rather limited by the solubilities of tetrazolium salts and the ultimately formed formazan and generally is less than 10 mM, with the lower limit set by the amount of reduced chromogen necessary to give a clear and distinct color signal, generally greater than 0.05 mM. Again, it should be noted, the invention is not limited to particular absolute values.

Other tetrazolium salts are disclosed in U.S. patents 4,490,465; 4,491,631; 4,598,042; 4,351,899; 4,271,265; 4,247,633; 4,223,090; 4,215,917; 4,142,938; 4,024,021; 3,867,259; 3,867,257; 3,791,931; and, 4,254,222.

To maintain the pH of the solution at a desired value during the reaction, a conventional buffer solution is used. Examples of the buffer solution are found in U.S. patents 4,416,983; 4,592,996; 4,271,265; 3,867,259; 3,867,258; and, 4,254,722.

In accordance with the third embodiment, it is advantageous to provide the catalytic electron-transfer in the presence of a salt, which may be preferably an inorganic salt. Palladium salt is used to increase the catalytic ability of meldola blue. Other salts have equivalent usefulness. Of particular interest are organic ligands of metals like ferrocene (dicyclopentadienyl iron). The noble metals like platinum or palladium in catalytic amounts enhance the catalytic activity of the catalyst. Suitable are water-soluble salts of palladium, like palladium chloride. Virtually the concentration of any organic compound which is a substrate for a NAD(P)-linked dehydrogenase system can be determined in accordance with all three embodiments of the invention. Organic compounds of interest include sugars, carbohydrates, e.g. glucose; galactose; ketones; organic acids like malic acid, lactic acid, uric acid; alcohols like methanol, ethanol, iditol, sorbitol, inositol; aldehydes like formaldehyde, acetaldehyde; proteins, albumen, bilirubin, beta-hydroxybutyrate; nitrates; antigens (like hepatitis B surface antigen, antigen(s) of acquired immune deficiency syndrome, immune deficiency virus, and others), amino acids or nucleotide sequences; cholesterol, triglycerides, glycerol 3-phosphate; glycine lactate, maleate and various other organic substrates that are reactive to enzyme-catalyzed dehydrogenation or hydrogenation and others disclosed in the literature.

The invention is useful as described herein for the measurement and determination of the amount of an organic compound generally in a fluid sample, more commonly in a biological fluid sample. Of course, a very large number of such compounds are of interest including: carbohydrates -e.g. glucose, amino acids, proteins, alcohols, sugars, ketones. Illustrative are the following: biological fluids including serum, plasma, whole blood, urine, saliva, amniotic and cerebrospinal fluids, and semen.

The fluids are not limited to those obtained from humans but also include those obtained from other mammals in general, including, for example, bovine, porcine, equine, feline and canine fluids. The fluids also include those obtained from non-mammals such as fish.

In accordance with the invention as was discussed above, the affinity of the enzyme(s) for the respective substrates is a guide for the selection of the substrates and according which embodiment of the invention is sought to be used. A general method of determining the dissociation constant ("Ks") of a two substrate enzyme is found in Rose I.A., O,Connell E.L. and Letwins J., Bio. Chem , 249 , pps. 563-568 (1974). For additional information for determining the bonding constant, see Enzyme Kinetics, Plowan, K., McGraw Hill, 1972.

When the substrate is present for the determination of its concentration, the system also includes a specific dehydrogenase for the specific substrate. Dehydrogenases are of course known. Typical dehydrogenases are the following commercially available dehydrogenases or to be available dehydrogenases or any other dehydrogenase can be substituted for the dehydrogenase used in each specific example: glucose dehydrogenase, L-glutamic dehydrogenase, glyoxylate reductase, hydroxybutyrate dehydrogenase, polyol dehydrogenase, sorbitol dehydrogenase, myo-inositol dehydrogenase, isocitrate dehydrogenase, 2-ketoglutarate dehydrogenase, leucine dehydrogenase, lipoamide dehydrogenase, malic dehydrogenase, malic enzyme, succinate semialdehyde oxidoreductase, 5-10-methylenetetrahydrofolate dehydrogenase, NADH peroxidase, cytochrome C reductase, octopine dehydrogenase, 3-phosphoglycerate dehydrogenase, dihydropteridine reductase, pyruvate dehydrogenase, saccharopine dehydrogenase, uridine-5'-diphosphate dehydrogenase, xylulose reductase, 6-phosphogluconic dehydrogenase, alanine dehydrogenase, dihydrofolate reductase, glucose-6-phosphate dehydrogenase, hydroxyacyl CoA dehydrogenase, 1 acetate dehydrogenase, glycerophosphate dehydrogenase, glycerol dehydrogenase, glyceraldehyde-3-phosphate dehydrogenase, alcohol dehydrogenase, aldehyde dehydrogenase, alpha-hydroxysteroid dehydrogenase, beta-hydroxysteroid dehydrogenase, ferredoxin oxido reductase, formaldehyde dehydrogenase, formate dehydrogenase, fructose dehydrogenase, and galactose dehydrogenase and other dehydrogenases which fulfill the equivalent function.

In the case where the NAD(P)H is generated by the action of a specific dehydrogenase acting on its specific substrate, it may be advantageous to incorporate into the buffers molecules that will react with and trap, immobilize, react with, etc., the oxidized specific substrate. These compounds are generally amine containing compounds, for example tris, or glycine that can also be utilized for pH control. Compounds that are especially useful for this trapping function are those of the general class known as alpha-effect amines. Alpha-effect amines are those amine-containing compounds that contain an atom that has unpaired electrons adjacent to the amine functional group. Examples of alpha-effect amines that can be advantageously incorporated into this system are hydrazine, N-substituted hydrazines, hydroxylamine, and o-substituted hydroxylamines, especially those 0-substituted hydroxylamines that are stable to drying due to their high boiling nature and low vapor pressure. An especially suited compound of this class is carboxymethoxy amine.

This invention can also be used with any mixture of enzymes to assay a biological molecule providing one of the resulting products of this mixture of string of enzymes is NADH or NAD(P)H. The use of mixtures of enzymes in the assay of biological molecules or of enzymes is known to those skilled in the art. An example of the use of a mixture of enzymes that will result in the production of NAD(P)H in the measurement of glucose is hexokinase and glucose 6-phosphate dehydrogenase. In this system, hexokinase converts glucose into glucose-6-phosphate in the presence of ATP. The glucose 6-phosphate so generated is oxidized by glucose-6-phosphate dehydrogenase with the resulting production of NAD(P)H. There is a potentially unlimited number of different combinations of enzymes that can be put together by one skilled in the art so that a resulting product is either NADH or NAD(P)H. A list of such enzymes would be obvious to those skilled in the art of enzyme assays. This invention can be used with all such methods using a series of enzyme reactions which have as a product a NADH or NAD(P)H .

It is specifically noteworthy that this invention is also useful in the determination of the activity of any enzyme, or combination of enzymes, that result in the production of NAD(P)H. For example, the amount of the medically useful enzyme, lactate dehydrogenase can be determined by the rate at which color is produced in a system containing lactate, necessary buffer salts, the ingredient of the invention and an unknown amount of lactate dehydrogenase enzyme. The reduction in the amount of color that is generated in this mixture, as opposed to that generated by conventional mixtures, wherein one molecule of dye is produced per molecule of NAD(P)H produced, will result in an advantageous greater period of time when the color is in the linear, visible discernable range. An example of a combination of enzymes that can be advantageously assayed by use of this invention is amylase in the presence of maltose dehydrogenase, as is described in U.S. patent 4,427,771 cited above.

It is important to note that in accordance with the invention, the compound, the concentration which is sought to be determined, need not be itself a substrate for the dehydrogenase. It is sufficient that it be capable of, and generate, a substrate for the dehydrogenase, for instance a triglyceride.

The process of the three embodiments comprises bringing together the necessary components of the system. The components or reactants are conveniently in an appropriate medium such as a liquid (e.g. aqueous) medium generally for most practical embodiments in or on an appropriate physical support. Such physical supports are known in the literature.

The assay system of the invention is provided in a variety of physical embodiments, including test kits and strips. Typically a test kit will include all the reagents described above and the sample is added to the reagents, which may be in a liquid system or a physical (solid) system like filter paper, etc. The competing substrate may be added to the system together with the test substance.

Typically, a test strip will be prepared by impregnating an absorbent material with solutions containing the reagents necessary for the corresponding determination. Suitable absorbent carriers for the test strips of the invention include all those inert absorbent carriers customarily in use for such tests. Most widespread is the utilization of filter paper, but other absorbent cellulose or synthetic resin products can likewise be employed.

Typically, test vials, as is known can be used. The device of the invention, which is often discardable after the threshold color has (or has not) developed, comprises a support member bearing a single or a plurality of concentration of the reactant. The support member is not critical in the sense that a specific material of construction is required although several forms of the preferred embodiment will be described hereinafter. In general, the support member may be of any material capable of bearing the reactant and the other components of the system, if necessary, for exposure to the solution to be tested. Specific examples of support members are webs, sticks, strips, splinters, sheets, filters, rods and like forms of glass, metal, wood, paper; polymerics such as polyethylene, polypropylene, polyalkylene acetate, polycarbonates and the like; gelatin and like materials; textiles and the like. Preferred materials are the bibulous materials which may be impregnated with solutions of reactant compositions, such as filter papers, blotting papers and like bibulous materials.

In one embodiment of the invention, the system can be incorporated into a bibulous material that is capable of taking up, by hydration or capillary action or other similar means, a set and reproducible amount of liquid. Materials that meet these criteria can be manufactured from a wide variety of solids. Examples of solid supports are: metal oxide, as typified by Norton Corporation's (Worcester, MA) controlled pore alumina HSA catalytic rings; polymeric materials, as typified by Nucleopore controlled-pore polycarbonate membranes; and hybrid ceramic/polymer materials as typified by Amerace's polyvinyl-chloride sheets that contain embedded silica particles. Such hybrid sheets are described in U.S. patent 4,169,014, which describes the coupling of active enzymes to these sheets. This patent does not address the utility of these sheets containing said active enzymes in containing a known and calibrated volume of liquid material.

In another ideal embodiment of the invention, the ingredients of the system may be incorporated into a multilayer dry gel which is situated inside a controlled volume capillary. When aqueous sample is introduced into the capillary, the gel, which may be made of any organic or synthetic polymer, like: gelatin, agarose, agar, polyvinyl alcohol, polyvinyl pyrrolidone, alginate, carrageenan, dextran, xanthan gum, or mixtures of the above, swells and rehydrates, activating the dried system ingredients. A non-obvious advantage of this system is that various ingredients of the system, for example the reactant and various buffer salts needed to stabilize a dehydrogenase enzyme, do not have to see one another (or be in contact with each other) during long term storage. Thus, storage incompatibilities of system ingredients is not encountered in multilayer systems.

The invention is not limited to the particular physical embodiments described above. There are numerous appropriate physical arrangements that are described in the literature and others can be built by one of average skill in the art.

The reactant components of the assay system of the invention are preferably prepared in a liquid form for a deposit upon the support member. Once placed on the support member, the reactant compositions in solution are dried to adhere the compositions to the support member. Generally, adhesion of the reactant compositions to the support member is conveniently effected when the support member is a bibulous material. Conventionally employed inert filters, binders, surfactants and the like may be incorporated into the reagent compositions when desired. Certain binders such as resin gums are advantageously incorporated into the reactant composition to assist in adhering them to non-porous support members such as metal, glass or non-porous polymeric materials. For product elegance and accuracy, it is desirable that the color change in each indicator zone of the devices of the invention be clear, sharp, unequivocal and strongly positive.

The device may be used as disclosed in U.S. patent 4,059,407 by immersion in the biological solution to be tested as for instance in a capillary. When it is desired that the device function in a thermometer-like fashion, the device is adapted to have several sites or regions. The device may be constructed as disclosed in said 4,059,407 patent. Reference is also made to its Figures which illustrate suitable physical embodiments of the present invention.

A more detailed description of the figures follows.

Fig. 1 shows the different color intensities generated by potassium ferricyanide in different concentrations in a reaction with diaphorase. Like other compounds disclosed on the first and second embodiments of the specification, potassium ferricyanide is not a "random access" substrate for diaphorase but one that is reacted preferentially over the chromogen.

Fig. 2 shows a similar behavior for benzoquinone.

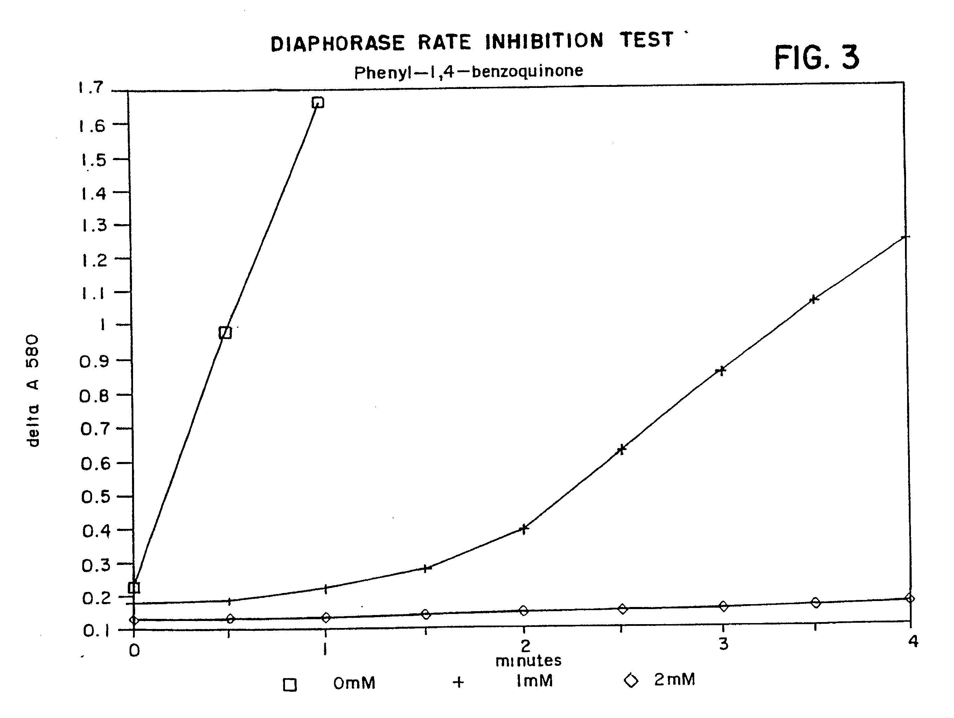

Fig. 3 likewise shows the color generated by the reaction of phenyl-1,4-benzoquinone by diaphorase at different concentrations of the benzoquinone.

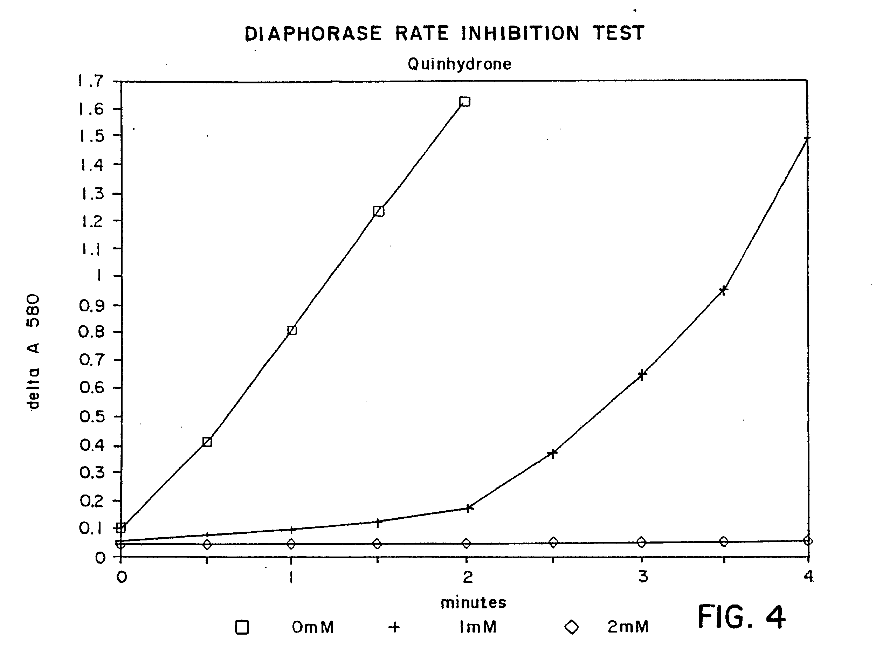

Fig. 4 shows a similar behavior by quinhydrone.

In Figs. 1 through 4 the respective substrates are preferentially reacted with respect to the MTT chromogen also present in the system.

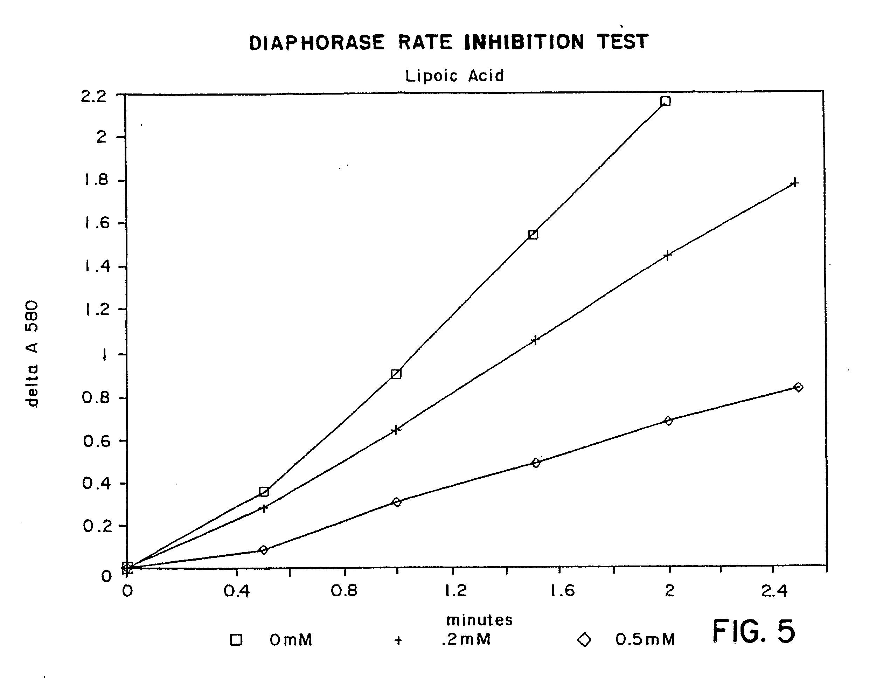

Fig. 5 shows the color produced in the reaction medium which contained 100 mM of potassium phosphate (pH 6.5) buffer, 1 mM MTT chromogen, 0.5 mM NADH, pig heart diaphorase, and a specific amount of lipoic acid as shown. The inhibition of the reaction by lipoic acid at different concentrations is clearly evident.

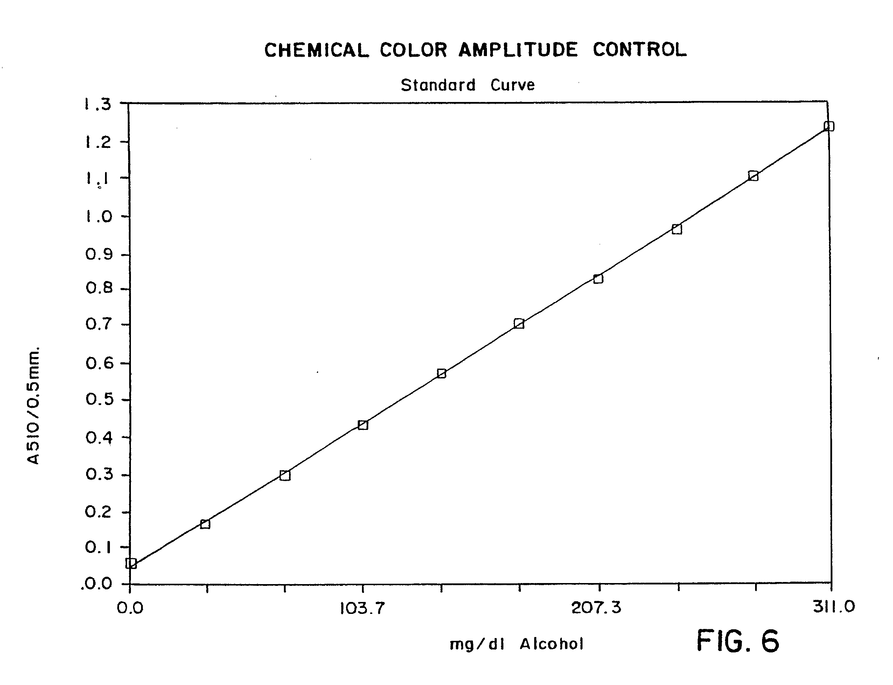

Fig. 6 is an illustration of the use of the invention for the determination of the amount of alcohol in saliva. Saliva samples are assayed in a solution containing 400 mM of lipoic acid, 5 mM of INT, 200 mM of potassium phosphate, NAD, 100 mM of semi-carbazide, 400 IU/ml of alcohol dehydrogenase and 150 IU/ml of diaphorase from microorganisms (commercially available from Toyo-Joyo [Japan] or Boehringer Mannheim, Indianapolis, Indiana); the final pH is 6.4.

A sample of saliva containing an unknown amount of alcohol is assayed and the content of alcohol determined by comparison with the standard curve as shown in Fig. 6.

Likewise another biological sample such as serum, containing alcohol in an unknown amount is assayed and compared to the standard curve.

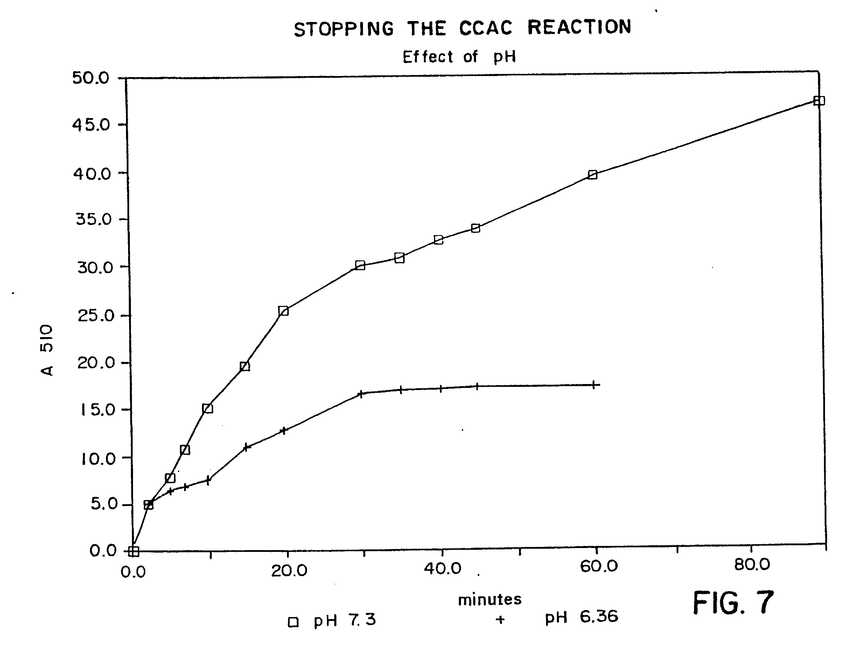

Fig. 7 is an illustration of a method for making the reaction of the secondary substrate irreversible, in this case that of lipoic acid. In Fig. 7 the reaction is stopped by lowering the pH in the presence of high concentrations of lipoic acid.

In Fig. 7 the reaction mixture contains 75 mM of lipoic acid, phosphate buffer, 5 mM INT, diaphorase, 40 mM NADH. At a pH of 6.36, the color development comes to a complete stop. At a pH of 7.3, the reaction of the lipoic acid is reversible and continues.

Therefore, in accordance with the invention, a practical method for making the reaction irreversible is to lower the pH to a pH at which the reaction would not proceed any further. Such pH threshold will be a pH lower than approximately 7.0.

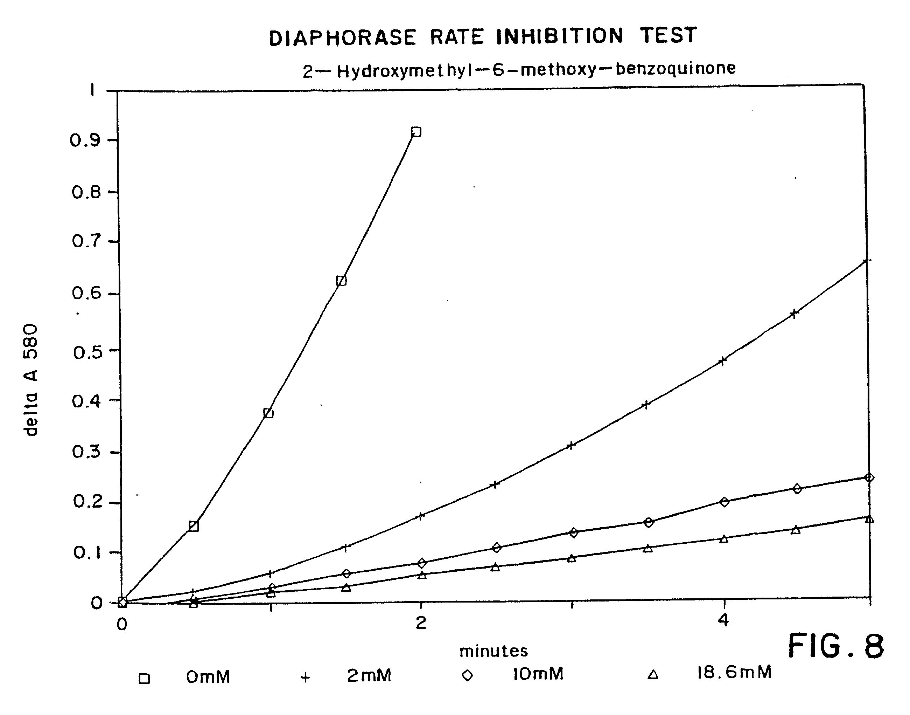

Fig. 8 is another illustration of the color development of a compound of the invention, showing color development in the presence of a chromogen, in this case MTT. Also shown is the use of a competing substrate 2-hydromethyl-6-methoxy benzoquinone. This competing substrate inhibits the reaction in the same manner as the lipoic acid.

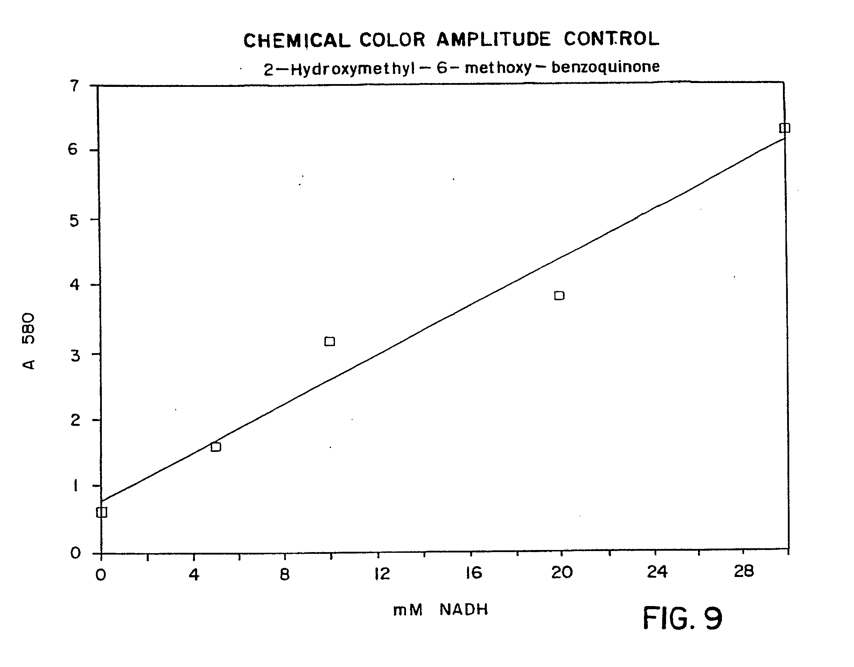

Fig. 9 is another illustration of the color that was produced in the presence of 4 mM of MTT, 100 mM HMMBQ in a mixture in a reaction system containing pig heart, diaphorase and a pH 7 phosphate buffer. It is noteworthy that the color generated at 40 mM of NADH in this system has an absorbence of 6 at 580 nm. In the absence of HMMBQ, the color that would not be obtainable as the solubility of MTT is only 20 mM, so that 40 mM of NADH cannot be colorimetrically measured using existing technology.

Another illustration of a compound suitable for use in the invention is diiodo-4-pyridone-N-acetic acid (DIPAA), commercially available from Aldrich Chemical Company, St. Louis, Missouri. In a system containing the following: 100 mM of DIPAA, 4 mM MTT (pH 74 phosphate buffer), pig heart, diaphorase and this mixture is reacted with 10 mM of NADH, the color of 62 absorbence units at 580 nm is obtained. In the absence of this DIPAA, the color would have been 240 absorbence units, therefore 3.9 times more than was observed.

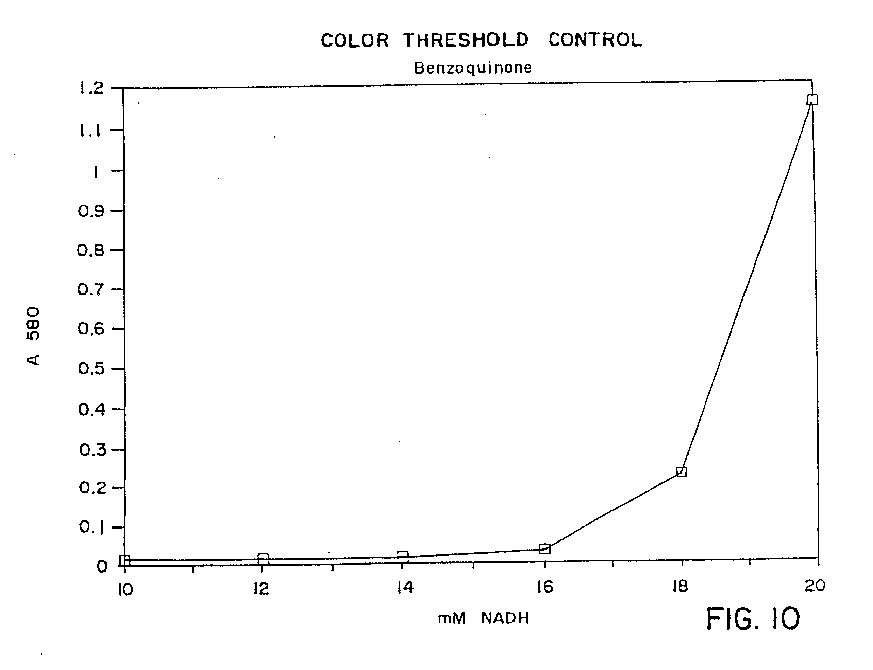

Fig. 10 is an illustration of a color-generating system containing 200 mM phosphate buffer (pH 7.3), microorganism diaphorase, 18 mM of benzoquinone and 1 mM of MTT. Concentrations of NADH from 2 mM to 20 mM are added to this reaction. As shown the figure, no color is generated as long as the concentrations of the NADH is less than 18 mM. When the concentration is greater than this pre-set threshold, the system turns to a very dark blue color. This embodiment of the invention comprises a second substrate (other than the chromogen) which is a preferential substrate for the diaphorase. In such a system the concentration of any given compound is determined at the pre-set threshold. When the color is below that pre-set threshold no color is generated. when the concentration of the test material is greater than the pre-set threshold than a color is generated. The threshold can be pre-set at any desired level. As shown in Fig. 10, the threshold is sharp and clear.

This system is very useful for monitoring concentrations of a substance in a solution. An illustration of this embodiment is a measuring device that is colorless in the presence of saliva alcohol less than the legal limit of 0.1% (i.e. 22 mM) and which develops color in the presence of saliva alcohol above that threshold level.

In a practical application of this embodiment, a device has all the necessary ingredients incorporated in a film which is placed over a blue printed sign such as "OK". At all concentrations of saliva alcohol measured which are less than 0.1%, the system remains pale yellow and the "OK" sign remains clearly visible. At concentrations of saliva alcohol above the cut-off of 0.1%, the system becomes dark blue and the "OK" sign would no longer be visible, thus alerting the person that saliva alcohol greater than the legal limit is present.

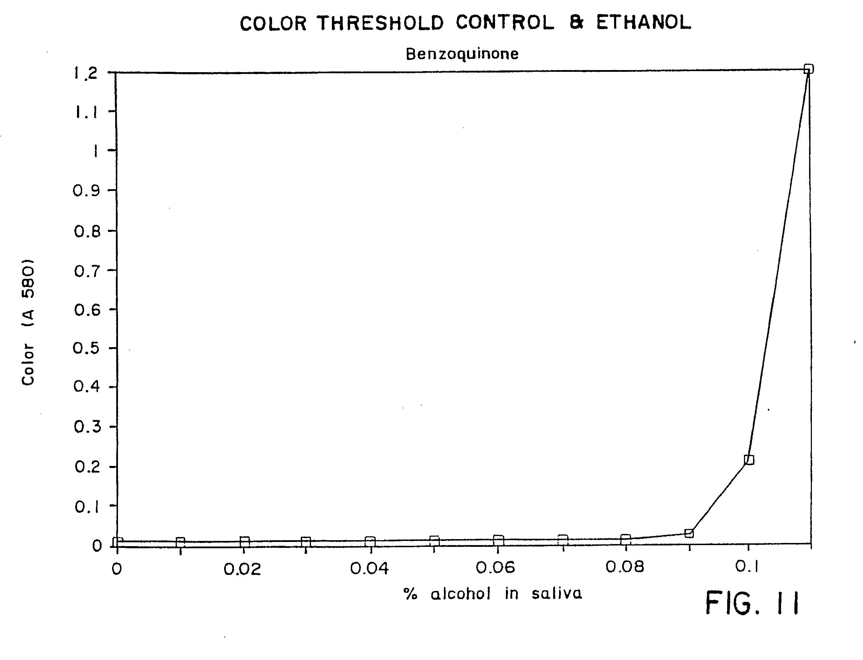

Fig. 11 is an illustration of measurements made in a kit which includes a clear gelatin-based film dried from a solution containing 21 mM benzoquinone, 1 mM MTT, 2 mM NAD, 15 mg/ml BSA, 100-1,000 IU/ml of alcohol dehydrogenase, 50-500 IU/ml diaphorase, 100 mM ph 7.7 tris-buffer, and 20mM 1,4-diaminobenzene.

The film is positioned in a controlled volume capillary which will contain 10 microliter of fluid.

A persons saliva is tested by introducing it into the capillary, the reaction color remains faint yellow until a concentration of 0.1% alcohol or greater is reached; the film then turns dark blue.

Of course, a threshold different from 0.1% of the sample (here alcohol) can be determined.

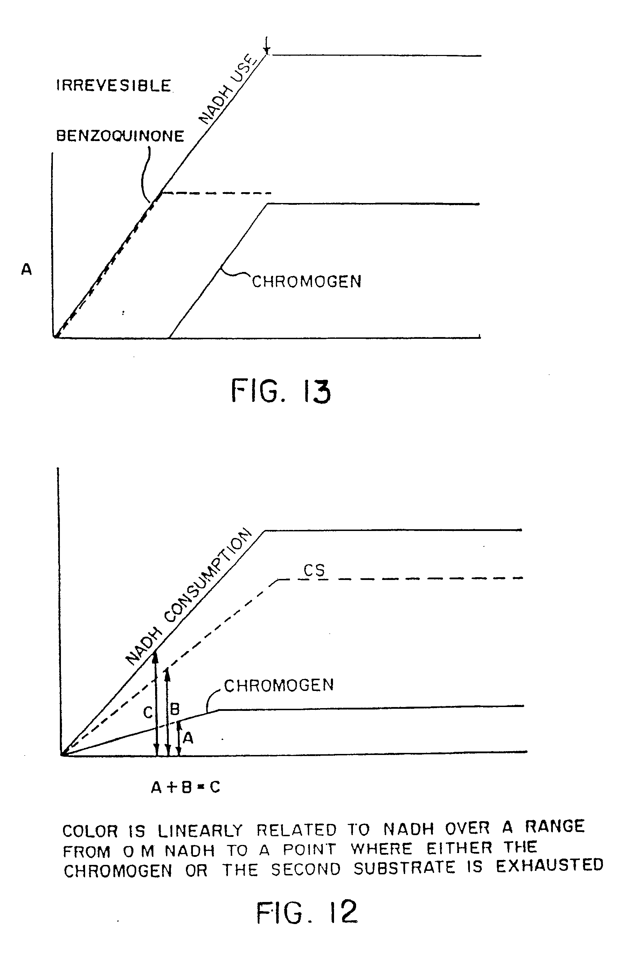

Fig. 12 is a graphic representation of the color generated and linearly related to NADH over a range from 7mM of NADH to a point where either the chromogen or the competing substrate is exhausted.

Fig. 13 is an illustration of the reaction of benzoquinone in presence of NADH showing the development of color due to the reaction of benzoquinone before that developed by the chromogen.

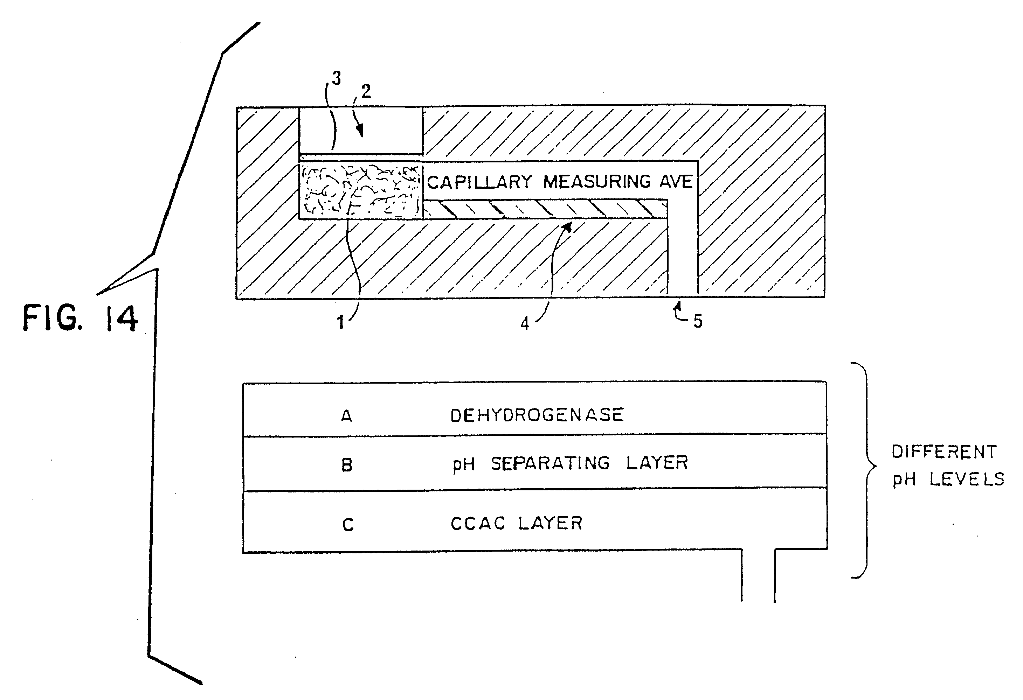

Fig. 14 is a diagrammatic representation of a physical embodiment of a test kit of the invention wherein 1 shows a wick where the sample is supplied, 2 shows a well for the sample separated by membrane 3 which will prevent passage of cells or other components in the reaction zone, the sample is reacted on the film 4 which is constituted by a layer A impregnated with dehydrogenase, and a layer C impregnated with the constituents of the Chemical Color Amplitude Control (CACC) system, separated by a layer B to separate the layers A and C which may be at different pHs for optimizing the activity of the exertive enzyme system. An air escape hole 5 is provided to allow for the venting of any gaseous products that may be generated.

Although the present invention has been described and illustrated in connection with preferred embodiments, it is to be understood that modifications and variations may be resorted to without departing from the spirit of the invention, as those skilled in the art will readily understand. Such modifications are considered to be within the purview and scope of the present invention as defined by the appended claims.

The invention having been described adequately to one skilled in the art to make use of it to the extent desired, the following examples are merely illustrative of the invention, and in no way are to be construed as limiting the invention. It is evident that without undue experimentation one skilled in this art can make many substitutions or variations and still be within the scope of the invention and obtain substantially the same results without departing from the teaching and spirit of the invention.

All parts are by weight unless indicated otherwise.

EXAMPLE 1

Colorimetric Assay of Alcohol in Liquid Solution

100 microliter of saliva are added to 100 microliter of the solution described below so that the resulting 200 microliter will have a final concentration as follows.

The solution is prepared of the following components. 200 mM lipoic acid is prepared by addition of 4M lipoic acid dissolved in 100% Triton X-100; 2% polyethylene glycol (1,000 molecular weight); 80 mM potassium phosphate monobasic; 120 mM potassium phosphate (dibasic); 100 mM semicarbazide, (from a 800 mM, pH 7.3 stock solution); 100 mM NAD; 2 mM INT; and 3 mg BSA.

To the solution there is added: 100 international units (IU) per ml of alcohol dehydrogenase (from yeast, Sigma Chemical Company, St. Louis, Missouri); and, 80 IU/ml diaphorase from microorganism (Boehringer Mannheim Corporation, New York, New York).

The contents of the tubes are mixed and left to react for five minutes. A standard curve is prepared by use with saliva that contains ethanol concentrations between 0 and 75 mM ethanol. After a five minute reaction time, the samples are read either directly in a 0.1 cm path length cuvette, or after a dilution in 50% dimethyl formamide.

Instead of saliva samples, serum samples from an individual can be used.

The absorbence that would have been seen in the absence of the lipoic acid at 75 mM ethanol is 675 absorbence units per cm, which is outside of the visible range.

Saliva samples are tested. The concentration of alcohol is determined to be 0.25, 0.1, 1.50, 7.0, 10.0, 12.0, 23 and 55 mM of ethanol when recorded against the standard curve.

The standard curve obtained from the reaction yields a straight line between 0.1 O.D. units per cm and 24 O.D. units per cm (0 and 1.2 O.D. units per 0.5mm) at 510 nm, for 0 and 75 mM ethanol, respectively.

EXAMPLE 2

Colorimetric Assay of Lactic Acid in Liquid Solution

100 microliter of serum or buffer containing lactic acid of between 0 and 25 mM is added to 100 microliter of a solution so that the final concentration will be as follows.

A mixture is prepared as follows: 150 mM lipoic acid is added at a pH of 7, tetramethylammonium chloride salt; 1.50 mM INT; 5 mg BSA; 200 mM pH 6.5 MES buffer; 40 mM hydrazine, (from a pH 7 stock); 0.2% Tween 80 ( a known wetting agent); 2 mM NAD; 100 IU/ml yeast lactate dehydrogenase (Sigma Chemical Company); and, 60 IU/ml pig liver lipoamide dehydrogenase (Sigma).

The reaction reaches a stable end point within one minute, at which time the color generated is read directly in a spectrophotometer in 1 cm or 0.1 cm path length cuvettes. The color generated by the standard curve yields a straight line between 0.1 and 8 absorbence units per cm at 510 nm. In the absence of lipoic acid, the amount of color generated at this wavelength would have been 180 absorbence units per cm.

The lipoic acid is replaced by 60 mM of DL-lipoamide beta-alanine. A like color intensity is obtained which can be read directly.

EXAMPLE 3

Assay of 0-150 mM Sorbitol in Liquid Solution

100 microliter of known or unknown sorbitol solutions that have a pH ranging between 4 and 10 are added to 100 microliter of a solution, so that the resulting solution has a composition as follows.

400 ml lipoic acid added at the pH of 7, tetramethyl-ammonium chloride salt; 150 mM oxidized 2-mercaptoethanol; 4% Triton X-100; 0.2% Tween 80; 8 mM BSA; 200 mM potassium phosphate pH 7.4 buffer; 150 mM semicarbazide; 5 mM NAD; 2 mM INT; 120 IU/ml sorbitol dehydrogenase or 150 IU/ml polyol dehydrogenase (Sigma); 60 IU/ml diaphorase from Clostridium kluyveri (Sigma or Genzyme, Boston, MA), from a 1,500 IU/ml stock that contained 0.5 mg/ml flavin adenine dinucleotide (FAD); and, 30 mM tris-Cl, pH 7.5 buffer.

The reaction is complete in one minute, at which time the sample is diluted 1/10 in 50% dimethyl formamide and read spectrophotometrically. The standard curve yields a straight line between 0.1 and 32 O.D. units at 510 nm for 0-150 mM of sorbitol. The absorbence seen at this wavelength in the absence of lipoic acid would have been 1,350 absorbence units. In this example, the amount of color that was generated at every concentration of sorbitol was reduced by a factor of 42 and into the visible range, from that would have been seen utilizing pre-existing technology.

EXAMPLE 4

Using the same procedure as shown above, the chromogen is replaced by DCPIP (1 mM) which is a dye which becomes of a less intense color as it is reacted. The reaction medium also includes 100 mM of hydroxymethyl benzoquinone as the competing substrate, 500 mM of phosphate buffer (pH 7.2) and NAD(P)H is measured in concentrations up to 50 mM. The color decrease due to the dye comes within a colorimetrically readable range.

EXAMPLE 5

Assay of Beta-Hydroxy Butyrate (2HB) in Liquid Solution

100 microliter of human serum containing an unknown concentration of 2HB was mixed with 100 microliter of a solution so that the final mixture contained: 80 mM DL-lipoic acid, added from a 2M solution in Triton X-100; 150 mM HEPES buffer, pH 7.2; 35 mM divalent metal ion-chloride; 10 mM NAD; 1.0 mM MTT; 15 mM 1-6 hexanedihydrazine; 60 IU/ml beta-hydroxybutyrate dehydrogenase (Sigma); and, 50 IU/ml Torula yeast lipoamine dehydrogenase (Sigma).

A biological sample was determined to contain 45 mM of 2HB.

A straight curve is also obtained by using concentrations of 2HB between 0 and 25 mM. The resulting reaction is complete within one minute. The color generated in this reaction reaches a high of 15 absorbence units at 580 nm. In the absence of the lipoic acid, the color generated would have reached 300 absorbence units.

A sample of human serum is determined to contain 15 mM of 2HB following the above procedure.

EXAMPLE 6

Production of a Dry Film Layer Containing the Chemical Color Amplitude Control System

Suitable films for diagnostic tests are prepared by different procedures. Three are illustrated below.

Method 1: To the cavity in a well washed glass or plastic plate containing a cavity that is approximately 0.5 inch in diameter and approximately 0.5 mm deep is added 20 microliter of a solution containing the following: 6% gelatin; 1.5% polyethylene glycol of molecular weight equal to or greater than 1,000; 20% sorbitol; 100 mM 1-4 butanedihydrazine; 100 mM MES buffer, pH 6.5; 0.2% Tween 80; 15 mg/ml BSA; 150 mM lipoic acid (as the pH 6.5 tetramethylammonium salt); 1.25 mM INT; and, 100 IU/ml pig heart lipoamide dehydrogenase.

The solution is gently heated to 37° C, and applied to the wells. The wells are then cooled to allow the gelatin to gel. Then the film is air dried by passing a stream of warm air over the film. Alternatively, the film is freeze dried under vacuum. When the films are dry to the touch, they are placed in a chamber and subjected to high vacuum for a period of time between 5 minutes and 30 minutes, sufficient to remove remaining traces of water. The films so generated are stored in the presence of a desiccant at temperatures below 30° C. Under these conditions, the films are stable for months.

These films are treated for operation of the Chemical Color Amplitude Control (CCAC) system by development with solutions containing between 0 and 100 mM of NAD(P)H.

In method (a) the polyethylene glycol is omitted; so is the butanedihydrazine, and the sorbitol (the latter normally added if enzyme stability needs to be increased). Likewise the Tween 80 can be omitted. The films which are obtained are suitable for use in the invention.

Method 2: To a clean test tube is added: 30 microliter 0.91 M MES buffer, pH 6.7; 12.5 microliter 350 mg/ml BSA; 60 microliter 10 mM INT; 15 microliter 200 mM NAD; 40 microliter 3,000 IU/ml diaphorase isolated from microorganisms (Boehringer Mannheim); 22.5 microliter 1.0 M lipoic acid at pH 6.7 tetramethylammonium salt; and, 72 microliter 25% gelatin warmed to 40° C.

The mixture is kept at a temperature sufficiently high so as to prevent gelling, then sprayed onto a sheet of clear plastic to an even 0.1 mm thickness. The sheet is dried in air and cut into even sized squares for later use.

Method 3: To a clean test tube are added: 12.5 microliter MES pH 6.5 buffer; 12.5 microliter 350 mg/ml BSA; 60 microliter INT; 15 microliter 200 mM NAD; 20 microliter 1,000 IU/ml pig heart lipoamide dehydrogenase; 22.5 microliter 1.0 M lipoic acid at pH 7, tetramethylammonium salt; 20 microliter 80% sorbitol; 58 microliter water; 78 microliter 25% gelatin; and, 10 microliter of either 1.0 M zinc sulfate or 1.0 M iodoacetic acid.

A continuous strip of plastic film is dipped into this solution, and the film then passed by a drying station which contains a warm air source or a radiant heat source. The film is then rolled onto a spool, and stored in a cool dry place until further use, or used immediately.

A similar strip is prepared using 22.5 microliter 1.0 M lipoic acid amidiated to 2-amino propanoic acid.

EXAMPLE 7

Production of a Multilayer Film for the Colorimetric Determination of Alcohol

A film for use in the invention, prepared by one of the methods detailed above, is used as the starting point in this production. The film is then covered with a very thin layer of gelatin (1 microliter, applied by spraying) which contains: 6% gelatin; 0.1% Triton X-100; and 30 mM pH 7.4 buffer.

This thin film is allowed to gel, then the well is covered with another layer containing: 140 mM potassium phosphate; 80 mM semicarbazide; 10 mM NAD; 15 mg/ml BSA; 0.5% Tween 80; 40% sorbitol; 6% gelatin; and 100 IU/ml alcohol dehydrogenase.

The sandwich so formed is cooled so that all layers will gel. The sandwich is then frozen and treated with high vacuum to remove moisture. This stable sandwich will reconstitute to generate a gel that turns color in the presence of aqueous ethanol or methanol. The color generated by the presence of each of these alcohols is compared to a chart (color calibrated). The intensity of the color corresponds to the concentration of the ethanol.

A number of blood samples are taken from patients and determined to contain varying concentrations of alcohol.

Concentrations are determined by comparison with a standard color scale.

EXAMPLE 8

Filter Paper impregnated with the Chemical Color Control System and Alcohol Dehydrogenase for Production of an Alcohol Saliva Diagnostic

A solution is prepared that contains: 200 mm potassium phosphate, pH 6.6; 120 mM semicarbazide, pH 6.6; 1 mM NAD, 15 mg/ml BSA; 4 mM INT, 120 mM lipoic acid, 4.5% sorbitol; 200 IU/ml lipoic acid dehydrogenase from yeast; and 20,000 IU/ml alcohol dehydrogenase.

A cellulose filter paper (for example Whatman #42), on a continuous roll is passed over a series of rollers. One of the rollers is positioned so as to dip the paper into the solution described. Immediately after the paper is dipped, the paper is lead past a drying station where it is well-dried by the action of hot air. The dry paper is then dipped into another solution that contains: gelatin 0-6%, in this sample about 3%; pH 6.6 phosphate buffer 2-200 mM, in this sample about 50 mM; Tween 80 about 0.1%; polystyrene at about 0.5%, (which has been finely dispersed in the semi-aqueous solution by sonic action); optionally there can be used other suitable agents that will contribute to the stabilization to the enzymes.

The wetted paper is again air dried, and the dried paper is passed over rollers onto a take-up roll. The paper is then processed by feeding to an automated cutter that will section the paper into small uniform sections of approximately 0.24 cm per side. Each section is then welded into a plastic, uniform volume capillary. The necessary wicking material and membranes that will draw sample to the measuring paper and prevent the passage to cells to this measuring paper are included in the final assembly. The final assembly has the components shown in Figure 14 following drawing.

In the kit described above (Fig. 14), the capillary layer also contains a non-ionic wetting agent to facilitate the spread of the substrate. The dehydrogenase layer A and the CCAC layer can be one single layer. A separation layer B is useful to avoid that resistance from the dehydrogenase reaction other than the desired oxidized substrate and NAD(P)H interfere with the color development test in the CCAC layer and acts thus as a trap (e.g. inhibitors of the diaphorase). Other variants of this construction can of course be considered.

EXAMPLE 9

A reaction mixture containing:
100 mM Tris buffer pH 9
21 mM NAD
1 mM MTT chromogen
1.25 mM meldola blue
0.1 mM palladium (II) chloride
100 IU/ml alcohol dehydrogenase
40 mM potassium ferricyanide
is treated with various concentrations of alcohol. It is found that this reaction is light grey when 18 mM alcohol is added, and dark blue when 22 mM alcohol is added.

EXAMPLE 10

To a reaction containing:
200 mM phosphate buffer pH 7.6
1 mM MTT
1.5 mM PMS
0.1 mM ferrocene
10 mM dimethylperoxide
is added various concentrations of NAD(P)H. It is found that the reaction remains essentially colorless when up to about 10 mM of NAD(P)H is added. When concentrations of NAD(P)H greater than 10 mM are added, the reaction turns bright blue.

EXAMPLE 11

To a reaction containing:
50 mM Tris buffer pH 7.3
1 mM MTT

1 mM PMS
6 mM NAD
12 mM ferricyanide
100 IU/ml lactic dehydrogenase
is added various concentration of lactic acid pH 7 solution. It is found that the reactions remain faint yellow when less than 6 mM of lactic acid is added, but turns bright blue when concentrations of lactic acid greater than this threshold are added.

EXAMPLE 12

An absorbent paper is soaked with a reaction containing:
300 mM Tris-chloride pH 9.0
21 mM NAD
40 mM potassium ferricyanide
1 mM PMS
1 mM MTT
100 IU/ml alcohol dehydrogenase
and the paper is dried and cut into 0.5 inch diameter circles. 20 microliter of solution containing various concentration of alcohol are added to each circle. It is found that circles exposed to 15 and 18 mM alcohol remain pale yellow, while circles exposed to 20 and 22 mM alcohol turn bright blue.

As discussed above, the invention can also be used to determine the concentration of molecules that are not themselves substrates for a dehydrogenase, as long as they can be acted upon to become dehydrogenase substrates. The following examples demonstrate this embodiment. In the case where triglyceride levels are measured after by conversion to glycerol, which is then oxidized by glycerol dehydrogenase to form NAD(P)H.

EXAMPLE 13

For the determination of triglyceride in an aqueous sample, for example serum, place down a 1-4 mil thick (wet) layer of 5-35% gelatin containing:
10 - 800 IU/ml glycerol dehydrogenase
50 - 1600 mM Tris base pH 9.0
10 mM NAD
0.02% triton X-704
0.1% palladium (II) chloride
1 mM PMS
This layer is overlaid with a separating layer that contains only gelatin or gelatin and other gelling agents and/or detergents. Another layer is layed down on top of the above that contains various linearly related step gradient concentrations, for example from 0 to 45 mM of either potassium or sodium ferricyanide or Fe(III)/EDTA reactant plus approximately 0.75 mM of chromogen, e.g. MTT. The above sandwich is overlaid with another separating layer as discussed above, then finally the entire stack is overlaid with a spreading layer that will cleanly wick the aqueous sample over the entire measuring area, such as:

5% microcrystalline cellulose

2.5% gelatin

0.03% Triton X-100

200 IU/ml lipase

50 IU/ml protease, for example alpha-chymotrypsin

The entire multilayer gel is dried in hot (37° C), dry air. The dry gel package is placed inside a capillary which will contain a set and reproducible volume of liquid sample per unit area of measuring surface.

Upon introduction of an aqueous sample of triglyceride, the lipase and protease hydrolyze the triglyceride to produce free glycerol. The free glycerol diffuses in the hydrated gel to the glycerol dehydrogenase whereon it is oxidized to produce NAD(P)H, which reacts with the reactant or the chromogen, as the case may be, to indicate the concentration of triglyceride in the sample.

As an alternative to the above, glycerol dehydrogenase may be substituted for by the combination of ATP, glycerol kinase, and glycerol phosphate dehydrogenase, or other suitable combinations as will be readily apparent to those with average skill in the art.

The system of the invention as noted above will normally contain as is known, various buffers compatible with the enzymes, stabilizers (for the enzymes on the resulting dyes) and, if desired, wetting agents. Illustrations are BSA, polyalcohols, mild reducing agents, non-ionic wetting agents. The pH is generally in the range of about 4 to about 11 (being optimized for the different enzymes used). The optimum pH ranges for different enzymes, or some enzymes of different origins are known. For instance, amongst the dehydrogenases, alcohol dehydrogenase has an optimum pH of 9.0, lactic and dehydrogenase, an optimum pH of 8.0. Amongst the diaphorase that from pig's heart has an optimum pH of 6.0 that from microorganisms, a pH of 7.3. Thus one skilled in the art will find it advisable to adjust the environment wherein the enzymes are to be active (be it the filter paper strip, the test tube, or other liquid or solid medium, etc.) at the optimum pH or within or close to the optimum range or value. It should be noted, that in the case of the multilayer device discussed above, all layers do not have to be stored (or do not have) at the same pH, which is greatly advantageous in storage shelf life of the ingredients.

Also useful in the practice of the invention will be chemicals that form gels or films that permit storing the essential ingredients in a dry state and rehydrating in the presence of an aqueous solution and controlling color generation. For such known chemicals see U.S. Patent 4,556,634, column 4.

For the purpose of this invention, the "threshold" is defined to mean as color change state which is indicative of the concentration of NAD(P)H: when the concentration of NAD(P)H is less than a threshold, no color exists; when the concentration of NAD(P)H is above the threshold, the clear, sharp, unequivocal preferably strong colour change will be produced. Where the reduced chromogen is colorless, the reverse situation applies.

Prior art provides description of devices, often disposable, which may be used in the practice of this invention as such or in a modification of such devices. The reagents and the reactions (and their sequence) of the system of the invention is novel and unobvious. Reference for such devices is made to U.S. Patents Nos. 4,059,407; and the patents listed therein; 3,464,871; U.S. patent 3,992,158 and the later patent referring to this invention. These patents described a multilayer analytical element that will change color in the presence of an analyzed molecule. The technology discussed in these patents yields an analog color signal for an analog concentration in-put The device and analytical element described can be modified in light of and with knowledge of the present invention to create a digital color signal. U.S. PATENTS 3,485,587 and 3,164,534 also describe a device that could be modified with the invented technology to improve them to register a digital "on/off" type signal.

Patents which also have been considered in the preparation of this patent application include PCT Publication WO85/01747.

It is to be noted that it is within the scope of the invention to use more than one reactant (inert with respect to each other) which will be sequentially consumed by the NAD(P)H in accordance with the invention. Likewise there may be used more than one chromogen. Similarly, the system can be used to determine more than one organic compound (with their respective enzyme systems), which compounds may have different reactivity levels with respect to the other reactants.

It is within the scope of the invention also to use the method of the invention on a continuous basis in a device adapted to feed the reactants to a multiplicity of reaction zones for the reactions to take place and the color change indications to develop.

Such device is likely to be of particular interest for industrial purposes like monitoring the absence, presence or concentration of organic compounds.

The preceding examples can be repeated by substituting or modifying the generically or specifically described reactants and/or operating conditions of this invention for those used in the preceding examples.

The invention has been described in detail with particular reference to certain preferred embodiments thereof, but it will be understood that variations and modifications can be effected within the spirit and scope of the invention.

**Claims**

1. A colorimetric assay method which is designed to measure the concentration of NAD(P)H, NAD(P)$^+$, or a substrate of an enzyme which reacts to form or consume NAD(P)H which assay comprises exposing to NAD(P)H or NAD(P)$^+$ (1) a chromogen which is an electron-acceptor from NAD(P)H and which changes colour in a visible range when it is reduced by accepting these electrons, (2) a sufficient amount of reactant which is capable of accepting electrons and preventing a visible colour change due to the accumulation of reduced chromogen until a predetermined threshold amount of NAD(P)H is generated, which reactant does not undergo an indicative colour change upon accepting such electrons, and (3) an electron-carrier catalyst capable of transferring electrons from NAD(P)H to the chromogen.

2. The method of Claim 1 wherein the reactant is capable of accepting electrons 1) directly from NAD(P)H, or 2) from reduced chromogen, thereby regenerating reduced chromogen, or from both.

3. The method of Claim 2 wherein the reactant is capable of accepting electrons directly from NAD(P)H, preferentially over the chromogen.

4. The method of Claim 1, 2 or 3 wherein the reactant is a weak oxidizing agent which agent is more electron-accepting than NAD(P)H/NAD-(P)$^+$.

5. The method of Claim 4 wherein the reactant is a compound selected from the group consisting of triethyldiamine chloride salt of Fe(III), the Fe(III) citrate complex, the Fe(III) EDTA complex, the Fe(III) sorbitol complex, hexamino cobalt (III) chloride, potassium hexacyanocobaltate (III) and sodium hexanitrocobaltate (III) or from the group consisting of sodium potassium periodate, paranitrobenzyl disulfide, 2,2'-dithiobis-(pyridine-N-oxide) and 2,2'-dithiobis(4-tert-butyl-1-isopropyl imidazole) or from the group consisting of trichloromelamine, hydrogen peroxide, a lower alkyl peroxide and benzoyl peroxide or the reactant is a water-soluble alkali metal salt of ferricyanide.

6. The method of any one of the preceding claims wherein the catalyst is diaphorase and the reactant is not a substrate for the diaphorase.

7. The method of any one of Claims 1 to 5 wherein the catalyst is an organic polycyclic compound.

8. The method of Claim 7 additionally comprising the use of a metal salt which enhances the catalytic activity of the catalyst.

9. The method of Claim 8 wherein the catalyst is an organic ligand complex of the metal salt.

10. The method of any one of the preceding claims wherein the chromogen is a tetrazolium salt.

11. The method of any one of the preceding claims, which comprises the use of a dehydrogenase corresponding to a specific organic substrate to be measured and NAD(P)+.

12. The method of Claim 11, which also comprises the reaction of the organic substrate the concentration of which is to be measured.

13. The method of Claim 12 wherein the substrate is selected from the group consisting of glucose, alcohol, lactic acid, glycerol, ketone, beta-hydroxybutyrate or a compound which reacts to generate said substrate in situ and the method makes use of the dehydrogenase which corresponds to said substrate.

14. The method of any one of the preceding claims wherein the device has a series of regions, each region having a pre-selected concentration of electron-accepting reactant which corresponds to a different threshold concentration of NAD(P)H to be determined.

15. The method of any one of Claims 1 to 4 wherein the NAD(P)H-dependent reduction of the chromogen is catalyzed by diaphorase and the electron-accepting reactant is a competing substrate for the diaphorase catalyzed reduction of the chromogen by NAD(P)H.

16. The method of Claim 15 wherein the assay further involves the use of means for causing the reduction of the competing substrate to be irreversible.

17. The method of Claim 16 wherein the said means is a second reactant for the reduced competing substrate.

18. The method of Claim 16 wherein the competing substrate is a lipoic acid compound and the means for causing the reaction to be irreversible is a compound that reacts preferentially or to a greater extent with the reduced lipoic acid compound as opposed to the oxidized lipoic acid compound.

19. The method of Claim 16 wherein the second reactant is selected from the group consisting of iodoacetic acid, oxidized 2-mercaptoethanol, chloroacetone, dichloro-acetone, methyl iodide, dibenzylsulfide, 2-hydroxy-methyl-6-methoxy-1,4-benzoquinone and diiodo-4-pyridone-N-acetic acid and the salts thereof or the second reactant is a chelating compound for the competing substrate.

20. The method of Claim 19 wherein the chelating compound is zinc, mercury, chromium or ferric ions.

21. The method of Claim 15 wherein the competing substrate is a lipoic acid compound, an anti-aromatic compound, a disulfide, a dithiobenzene, or a benzoquinone.

22. An assay method for colorimetrical measurement and determination of NAD(P)H, NAD(P) or a substrate of an enzyme which reacts to form or consume NAD(P)H which method comprises:
a diaphorase which catalyzes the NAD(P)H-dependent reduction of a chromogen, which is an electron acceptor, is a substrate for the diaphorase, and causes an indicative colour change when the chromogen is reduced by NAD(P)H, and
a competing substrate for the diaphorase which is an electron acceptor, is irreversibly reduced by the diaphorase and causes no indicative colour change when it accepts electrons from the diaphorase,
whereby the change in colour caused by reduction of the chromogen is in a ratio of less than one molecule of dye per equivalent of NAD(P)H produced and is indicative of the concentration sought to be determined.

23. A diagnostic assay method for the quantitative or qualitative determination of an organic sample in a biological medium, which comprises the use of a combination of support means for a dehydrogenase for the biological medium, a chromogen, NAD(P)H, a competing substrate which irreversibly is reducible by NAD(P)H in the presence of diaphorase and which competing substrate causes no indicative colour change upon reduction, and diaphorase,
which combination causes an indicative change of colour when the organic substrate is contacted with said combination, the organic sample is oxidized, and chromogen and the competing substrates are reduced.

24. The method of Claim 22 or 23 wherein the competing substrate is a lipoic acid compound.

25. A colorimetric device for performing the assay method of any of Claims 1 to 10 comprising the chromogen, the reactant and the catalyst.

26. A colorimetric device for performing the assay method of Claim 11 comprising the chromogen, the reactant, the catalyst, NAD(P)$^+$ and a dehydrogenase corresponding to the specific substrate to be measured.

27. A colorimetric device for performing the method of any one of Claims 16-21 comprising the chromogen, the reactant, the catalyst and the said means for causing the reduction of the competing substrate to be irreversible.

28. The device of any one of Claims 25 to 27 comprising a physical support means for the chromogen which is a water-absorbing material inert to the reactants, the physical support means being in a form selected from the group consisting of sheets, rods, webs, filters and strips.

29. The device of Claim 28, wherein the physical support means is glass, cellulose, wood, metal, textile, metal oxide, hybrid ceramic/polymer, chromogenic paper, gelatin or synthetic resin, or a polymeric from the group consisting of polyethylene, polypropylene, polyalkylene-acetate and polycarbonate.

30. The device of Claim 28 wherein the physical support means is situated inside a controlled volume capillary and is a multilayer dry gel such as gelatin, agarose, agar, polyvinyl alcohol, polyvinyl pyrrolidone, alginate, carrageenan, dextran, xanthan gum, or mixtures thereof.

## Revendications

1. Méthode d'essai colorimétrique, conçue pour mesurer la concentration de NAD(P)H, NAD(P)-$^+$, ou d'un substrat d'une enzyme qui réagit pour former ou consommer NAD(P)H, lequel essai consiste à exposer à NAD(P)H ou NAD-(P)$^+$ (1) un chromogène qui est un accepteur d'électrons en provenance de NAD(P)H et qui change de couleur dans une plage visible lorsqu'il est réduit par l'acceptation de ces électrons, (2) une quantité suffisante de réactif qui est susceptible d'accepter des électrons et d'empêcher un changement de couleur visible dû à l'accumulation du chromogène réduit jusqu'à la production d'une quantité de seuil prédéterminée de NAD(P)H, lequel réactif ne subit pas de changement de couleur indicatif en acceptant ces électrons, et (3) un catalyseur support d'électrons susceptible de transférer des électrons de NAD(P)H au chromogène.

2. Méthode selon la revendication 1, dans laquelle le réactif est susceptible d'accepter des électrons (1) directement de NAD(P)H, ou (2) du chromogène réduit, régénérant ainsi le chromogène réduit, ou des deux.

3. Méthode selon la revendication 2, dans laquelle le réactif est susceptible d'accepter des électrons directement de NAD(P)H, de préférence à ceux en provenance du chromogène.

4. Méthode selon l'une des revendications 1, 2 ou 3, dans laquelle le réactif est un agent d'oxydation faible, lequel agent est un meilleur accepteur d'électrons que NAD(P)H/ NAD(P)$^+$.

5. Méthode selon la revendication 4, dans laquelle le réactif est un composé choisi dans le groupe comprenant le triéthyldiamine chlorure de Fe (III), le complexe Fe(III) citrate, le complexe Fe(III) EDTA, le complexe Fe(III) sorbitol, le chlorure d'hexamino cobalt (III), l'hexacyanocobaltate (III) de potassium et l'hexanitrocobaltate (III) de sodium, ou dans le groupe comprenant le periodate de sodium potassium, le disulfure de paranitrobenzyle, le 2,2'-dithiobis-(pyridine-N-oxyde) et le 2,2'-dithiobis(4-tert-butyl-1-isopropyl imidazole), ou dans le groupe comprenant la trichloro mélamine, le peroxyde d'hydrogène, un peroxyde d'alcoyle inférieur et le peroxyde de benzoyle, ou dans laquelle le réactif est un sel de métal alcalin de ferricyanure soluble dans l'eau.

6. Méthode selon l'une quelconque des revendications précédentes, dans laquelle le catalyseur est une diaphorase et le réactif n'est pas un substrat pour la diaphorase.

7. Méthode selon l'une quelconque des revendications 1 à 5, dans laquelle le catalyseur est un composé polycyclique organique.

8. Méthode selon la revendication 7, consistant en outre à utiliser un sel métallique qui augmente l'activité catalytique du catalyseur.

9. Méthode selon la revendication 8, dans laquelle le catalyseur est un complexe d'un coordinat organique du sel métallique.

10. Méthode selon l'une quelconque des revendications précédentes, dans laquelle le chromogène est un sel de tétrazolium.

11. Méthode selon l'une quelconque des revendications précédentes, qui consiste à utiliser une déshydrogénase correspondant à un substrat organique spécifique à mesurer et à NAD(P)$^+$.

12. Méthode selon la revendication 11, qui consiste également à faire réagir le substrat organique dont la concentration est à mesurer.

13. Méthode selon la revendication 12, dans laquelle le substrat est choisi dans le groupe comprenant les substances suivantes : glucose, alcool, acide lactique, glycérol, cétone, béta-hydroxybutyrate ou un composé qui réagit pour produire ce substrat in situ, la méthode utilisant la déshydrogénase qui correspond à ce substrat.

14. Méthode selon l'une quelconque des revendications précédentes, dans laquelle le dispositif a une série de régions, chaque région ayant une concentration présélectionnée du réactif accepteur d'électrons qui correspond à une concentration de seuil différente du NAD(P)H à déterminer.

15. Méthode selon l'une quelconque des revendications 1 à 4, dans laquelle la réduction dépendant de NAD(P)H du chromogène est catalysée par la diaphorase et dans laquelle le réactif accepteur d'électrons est un substrat concurrent pour la réduction catalysée par la diaphorase du chromogène par NAD(P)H.

16. Méthode selon la revendication 15, dans laquelle l'essai implique en outre l'utilisation de moyens pour rendre irréversible la réduction du substrat concurrent.

17. Méthode selon la revendication 16, dans laquelle ces moyens sont constitués par un deuxième réactif pour le substrat concurrent réduit.

18. Méthode selon la revendication 16, dans laquelle le substrat concurrent est un composé d'acide lipoïque et les moyens pour rendre irréversible la réaction sont constitués par un composé qui réagit de préférence ou à un degré plus important avec le composé d'acide lipoïque réduit, par opposition au composé d'acide lipoïque oxydé.

19. Méthode selon la revendication 16, dans laquelle le deuxième réactif est choisi dans le groupe comprenant les substances suivantes : acide iodoacétique, 2-mercaptoéthanol oxydé, chloro acétone, dichloro acétone, iodure de méthyle, dibenzylsulfure, 2-hydroxy-méthyl-6-méthoxy-1,4-benzoquinone et acide diiodo-4-pyridone-N-acétique, et leurs sels, ou dans laquelle le deuxième réactif est un composé chélatant pour le substrat concurrent.

20. Méthode selon la revendication 19, dans laquelle le composé chélatant est le zinc, le mercure, le chrome ou des ions ferriques.

21. Méthode selon la revendication 15, dans laquelle le substrat concurrent est un composé d'acide lipoïque, un composé anti-aromatique, un disulfure, un dithiobenzène ou une benzoquinone.

22. Méthode d'essai pour la mesure colorimétrique et la détermination de NAD(P)H, NAD(P)$^+$ ou d'un susbtrat d'une enzyme qui réagit pour former ou consommer NAD(P)H, laquelle méthode comprend :
une diaphorase qui catalyse la réduction dépendant de NAD(P)H d'un chromogène, qui est un accepteur d'électrons, est un substrat pour la diaphorase et provoque un changement de couleur indicatif lorsque le chromogène est réduit par NAD(P)H, et
un substrat concurrent pour la diaphorase qui est un accepteur d'électrons, est déduit de façon irréversible par la diaphorase et ne provoque aucun changement de couleur indicatif lorsqu'il accepte des électrons de la diaphorase,
d'où il résulte que le changement de couleur provoqué par la réduction du chromogène se fait dans un rapport inférieur à une molécule de colorant par équivalent de NAD(P)H produit et est indicatif de la concentration que l'on cherche à déterminer.

23. Méthode d'essai diagnostique pour la détermination quantitative ou qualitative d'un échantillon organique dans un milieu biologique, qui consiste à utiliser une combinaison d'un support pour une déshydrogénase pour le milieu biologique, d'un chromogène, de NAD(P)H, d'un substrat concurrent qui peut être réduit de façon irréversible par NAD(P)H en présence de la diaphorase, lequel substrat concurrent ne provoque aucun changement de couleur indicatif lors de la réduction, et d'une diaphorase, laquelle combinaison provoque un changement indicatif de couleur lorsque le substrat organique est mis en contact avec cette combinaison, l'échantillon organique est oxydé, et le chromogène et le susbtrat concurrent sont réduits.

24. Méthode selon la revendication 22 ou la revendication 23, dans laquelle le susbtrat concurrent est un composé d'acide lipoïque.

25. Dispositif colorimétrique pour mettre en oeuvre la méthode d'essai selon l'une des revendications 1 à 10, comprenant le chromogène, le réactif et le catalyseur.

26. Dispositif colorimétrique pour mettre en oeuvre la méthode d'essai de la revendication 11, comprenant le chromogène, le réactif, le catalyseur, NAD(P)$^+$ et une déshydrogénase correspondant au substrat spécifique à mesurer.

27. Dispositif colorimétrique pour mettre en oeuvre la méthode selon l'une quelconque des revendications 16 à 21, comprenant le chromogène, le réactif, le catalyseur et les moyens pour rendre irréversible la réduction du substrat concurrent.

28. Dispositif selon l'une quelconque des revendications 25 à 27, comprenant un support physique pour le chromogène qui est un matériau absorbant l'eau inerte vis-à-vis des réactifs, le support physique se présentant sous une forme choisie dans le groupe comprenant des feuilles, des tiges, des nappes, des filtres et des bandes.

29. Dispositif selon la revendication 28, dans lequel le support physique est du verre, la cellulose, du bois, un métal, un textile, un oxyde métallique, un hybride polymère/céramique, un papier chromogène, de la gélatine ou une résine synthétique, ou un polymère choisi dans le groupe comprenant un polyéthylène, un polypropylène, un acétate de polyalcoylène et un polycarbonate.

**30.** Dispositif selon la revendication 28, dans lequel le support physique est situé à l'intérieur d'un volume capillaire contrôlé et est un gel sec multicouche, tel que gélatine, agarose, agar, alcool polyvinylique, polyvinyl pyrrolidone, alginate, carraghen, dextrane, gomme de xanthane ou leurs mélanges.

## Ansprüche

**1.** Kolorimetrisches Testverfahren, das zur Bestimmung der Konzentration von NAD(P)H, NAD(P)$^+$ oder eines Substrats eines unter Bildung oder Verbrauch von NAD(P)H reagierenden Enzyms bestimmt ist, durch Einwirkenlassen auf NAD(P)H oder NAD(P)$^+$

(1) eines Chromogens, bei dem es sich um einen Elektronen-akzeptor von NAD(P)H handelt und das bei der Reduktion durch Aufnahme dieser Elektronen die Farbe in einem sichtbaren Bereich ändert,

(2) einer ausreichenden Menge eines Reaktionsteilnehmers, der zur Elektronenaufnahme und Verhinderung einer sichtbaren Farbänderung infolge Ansammlung von reduziertem Chromogen bis zum Entstehen eines vorgegebenen Schwellenwerts von NAD(P)H fähig ist und der bei Aufnahme solcher Elektronen keine anzeigende Farbänderung erfährt, und

(3) eines zur Übertragung von Elektronen von NAD(P)H auf das Chromogen fähigen Elektronenträgerkatalysators.

**2.** Verfahren nach Anspruch 1, dadurch gekennzeichnet, daß der Reaktionsteilnehmer zur Aufnahme von Elektronen

1. direkt aus NAD(P)H oder

2. aus reduziertem Chromogen, unter Regenerierung von reduziertem Chromogen, oder aus beiden fähig ist.

**3.** Verfahren nach Anspruch 2, dadurch gekennzeichnet, daß der Reaktionsteilnehmer zur Aufnahme von Elektronen direkt aus NAD(P)H, vorzugsweise über das Chromogen, fähig ist.

**4.** Verfahren nach Anspruch 1, 2 oder 3, dadurch gekennzeichnet, daß es sich bei dem Reaktionsteilnehmer um ein schwaches Oxidationsmittel handelt, das leichter Elektronen aufnimmt als NAD(P)H/NAD(P)$^+$.

**5.** Verfahren nach Anspruch 4, dadurch gekennzeichnet, daß es sich bei dem Reaktionsteilnehmer um eine Verbindung aus der Gruppe: Triethyldiaminchloridsalz von Fe(III), Fe(III)-Citratkomplex, Fe(III)-EDTA-Komplex, Fe(III)-Sorbitkomplex, Hexaminokobalt(III)-chlorid, Kaliumhexacyanocobaltat(III) und Natriumhexanitrocobaltat(III) oder aus der Gruppe: Natriumkaliumperjodat, p-Nitrobenzyldisulfid, 2,2'-Dithiobis-(pyridin-N-oxid) und 2,2'-Dithiobis-(4-tert.-butyl-1-isopropylimidazol) oder aus der Gruppe: Trichlormelamin, Wasserstoffperoxid, Niedrigalkylperoxid und Benzoylperoxid handelt oder daß der Reaktionsteilnehmer aus einem wasserlöslichen Alkalimetallferricyaniacalz besteht.

**6.** Verfahren nach einem der vorhergehenden Ansprüche, dadurch gekennzeichnet, daß der Katalysator aus Diaphorase besteht und es sich bei dem Reaktionsteilnehmer nicht um ein Substrat für die Diaphorase handelt.

**7.** Verfahren nach einem der Ansprüche 1 bis 5, dadurch gekennzeichnet, daß es sich bei dem Katalysator um eine organische polycyclische Verbindung handelt.

**8.** Verfahren nach Anspruch 7, dadurch gekennzeichnet, daß zusätzlich ein Metallsalz mitverwendet wird, das die katalytische Aktivität des Katalysators verstärkt.

**9.** Verfahren nach Anspruch 8, dadurch gekennzeichnet, daß es sich bei dem Katalysator um einen organischen Ligandkomplex des Metallsalzes handelt.

**10.** Verfahren nach einem der vorhergehenden Ansprüche, dadurch gekennzeichnet, daß es sich bei dem Chromogen um ein Tetrazoliumsalz handelt.

**11.** Verfahren nach einem der vorhergehenden Ansprüche, dadurch gekennzeichnet, daß eine Dehydrogenase entsprechend einem zu bestimmenden speziellen organischen Substrat und NAD(P)$^+$ verwendet werden.

**12.** Verfahren nach Anspruch 11, bei dem auch die Reaktion des organischen Substrats, dessen Konzentration bestimmt werden soll, erfolgt.

**13.** Verfahren nach Anspruch 12, dadurch gekennzeichnet, daß das Substrat aus der Gruppe: Glucose, Alkohol, Milchsäure, Glycerin, Keton, ß-Hydroxybutyrat oder eine Verbindung, die unter Bildung des Substrats in situ reagiert, ausgewählt ist und das Verfahren sich der diesem Substrat entsprechenden Dehydrogenase bedient.

14. Verfahren nach einem der vorhergehenden Ansprüche, dadurch gekennzeichnet, daß das Hilfsmittel eine Reihe von Zonen aufweist, von denen jede eine vorher gewählte Konzentration an Elektronen aufnehmendem Reaktanten entsprechend einer zu ermittelnden unterschiedlichen Schwellenkonzentration an NAD(P)H aufweist.

15. Verfahren nach einem der Ansprüche 1 bis 4, dadurch gekennzeichnet, daß die NAD(P)H-abhängige Reduktion des Chromogens durch Diaphorase katalysiert wird und es sich bei dem Elektronen aufnehmenden Reaktanten um ein konkurrierendes Substrat für die diaphorasekatalysierte Reduktion des Chromogens durch NAD(P)H handelt.

16. Verfahren nach Anspruch 15, dadurch gekennzeichnet, daß zusätzlich von einem Mittel Gebrauch gemacht wird, das die Reduktion des konkurrierenden Substrats irreversibel macht.

17. Verfahren nach Anspruch 16, dadurch gekennzeichnet, daß es sich bei dem betreffenden Mittel um einen zweiten Reaktionsteilnehmer für das reduzierte konkurrierende Substrat handelt.

18. Verfahren nach Anspruch 16, dadurch gekennzeichnet, daß das konkurrierende Substrat aus einer Liponsäureverbindung besteht und es sich bei dem Mittel zum Irreversibelmachen der Reaktion um eine Verbindung handelt, die im Gegensatz zu der oxidierten Liponsäureverbindung vorzugsweise oder in höherem Ausmaß mit der reduzierten Liponsäureverbindung reagiert.

19. Verfahren nach Anspruch 16, dadurch gekennzeichnet, daß der zweite Reaktionsteilnehmer aus der Gruppe: Jodessigsäure, oxidiertes 2-Mercaptoethanol, Chloraceton, Dichloraceton, Methyljodid, Dibenzylsulfid, 2-Hydroxymethyl-6-methoxy-1,4-benzochinon und Dijod-4-pyridon-N-essigsäure und deren Salzen ausgewählt ist oder daß der zweite Reaktionsteilnehmer aus einem Chelatbildner für das konkurrierende Substrat besteht.

20. Verfahren nach Anspruch 19, dadurch gekennzeichnet, daß es sich bei dem Chelatbildner um Zink-, Quecksilber-, Chrom- oder Eisen(III)-ionen handelt.

21. Verfahren nach Anspruch 15, dadurch gekennzeichnet, daß es sich bei dem konkurrierenden Substrat um eine Liponsäureverbindung, eine antiaromatische Verbindung, ein Disulfid, ein Dithiobenzol oder ein Benzochinon handelt.

22. Testverfahren zur kolorimetrischen Messung und Bestimmung von NAD(P)H, NAD(P)$^+$ oder eines Substrats eines unter Bildung oder Verbrauch von NAD(P)H reagierenden Enzyms, umfassend:
eine Diaphorase, die die NAD(P)H-abhängige Reduktion eines Chromogens, das einen Elektronenakzeptor darstellt, ein Substrat für die Diaphorase bildet und bei seiner Reduktion durch NAD(P)H eine anzeigende Farbänderung herbeiführt, katalysiert, und
ein konkurrierendes Substrat für die Diaphorase, das einen Elektronenakzeptor darstellt, irreversibel durch die Diaphorase reduziert wird und bei der Aufnahme von Elektronen aus der Diaphorase keine anzeigende Farbänderung erfährt,
wobei die durch die Reduktion des Chromogens bedingte Änderung in der Färbung in einem Verhältnis von weniger als ein Molekül Farbstoff pro Äquivalent an gebildetem NAD(P)H erfolgt und als Anzeichen für die zu bestimmende Konzentration dient.

23. Diagnostisches Testverfahren zur quantitativen oder qualitativen Bestimmung einer organischen Probe in einem biologischen Medium, umfassend die Verwendung einer Kombination eines Trägers für eine Dehydrogenase für das biologische Medium, ein Chromogen, NAD(P)H, ein konkurrierendes Substrat, das in Gegenwart von Diaphorase irreversibel durch NAD(P)H reduzierbar ist und bei der Reduktion keine anzeigende Farbänderung herbeiführt, und Diaphorase,
wobei die Kombination eine anzeigende Änderung in der Färbung herbeiführt, wenn das organische Substrat mit der Kombination in Berührung gebracht, die organische Probe oxidiert und das Chromogen und die konkurrierenden Substrate reduziert werden.

24. Verfahren nach Anspruch 22 oder 23, dadurch gekennzeichnet, daß es sich bei dem konkurrierenden Substrat um eine Liponsäureverbindung handelt.

25. Kolorimetrisches Hilfsmittel zur Durchführung des Testverfahrens nach einem der Ansprüche 1 bis 10, umfassend das Chromogen, den Reaktionsteilnehmer und den Katalysator.

26. Kolorimetrisches Hilfsmittel zur Durchführung des Testverfahrens nach Anspruch 11, umfassend das Chromogen, den Reaktionsteilnehmer, den Katalysator, NAD(P)$^+$ und eine Dehydrogenase entsprechend dem zu bestimmenden speziellen Substrat.

27. Kolorimetrisches Hilfsmittel zur Durchführung des Verfahrens nach einem der Ansprüche 16 bis 21, umfassend das Chromogen, den Reaktionsteilnehmer, den Katalysator und das Mittel zum Irreversibelmachen der Reduktion des konkurrierenden Substrats.

28. Hilfsmittel nach einem der Ansprüche 25 bis 27, umfassend einen physikalischen Träger für das Chromogen, bei dem es sich um ein gegenüber den Reaktionsteilnehmern inertes wasserabsorbierendes Material handelt, in Folien-, Stäbchen-, Lagen-, Filter- oder Streifenform.

29. Hilfsmittel nach Anspruch 28, dadurch gekennzeichnet, daß der physikalische Träger aus Glas, Cellulose, Holz, Metall, textilem Material, Metalloxid, einem Keramik/ Polymerhybrid, chromogenem Papier, Gelatine oder einem Kunstharz, oder einem Polymeren aus der Gruppe: Polyethylen, Polypropylen, Polyalkylenacetat und Polycarbonat, besteht.

30. Hilfsmittel nach Anspruch 28, dadurch gekennzeichnet, daß sich der physikalische Träger im Inneren einer ein festgelegtes Volumen aufweisenden Kapillare befindet und daß er aus einem mehrschichtigen trockenen Gel, wie Gelatine, Agarose, Agar, Polyvinylalkohol, Polyvinylpyrrolidon, Alginat, Carrageenan, Dextran, Xanthangummi oder Mischungen derselben, besteht.

DIAPHORASE COMPETING SUBSTRATE TEST

Potassium Ferricyanide

FIG. I

FIG. 2

DIAPHORASE COMPETING SUBSTRATE TEST

Benzoquinone

FIG. 3

DIAPHORASE RATE INHIBITION TEST
Phenyl—1,4—benzoquinone

delta A 580

□ 0mM    + 1mM    ◇ 2mM

minutes

# DIAPHORASE RATE INHIBITION TEST

## Quinhydrone

FIG. 4

□ 0mM　　+ 1mM　　◇ 2mM

EP 0 279 988 B1

DIAPHORASE RATE INHIBITION TEST

Lipoic Acid

FIG. 5

□ 0mM    + .2mM    ◇ 0.5mM

## CHEMICAL COLOR AMPLITUDE CONTROL

Standard Curve

FIG. 6

EP 0 279 988 B1

# STOPPING THE CCAC REACTION
## Effect of pH

FIG. 7

□ pH 7.3          + pH 6.36

DIAPHORASE RATE INHIBITION TEST

2— Hydroxymethyl—6—methoxy—benzoquinone

FIG. 8

| □ | 0mM | + | 2mM | ◇ | 10mM | △ | 18.6mM |

EP 0 279 988 B1

CHEMICAL COLOR AMPLITUDE CONTROL

2−Hydroxymethyl − 6− methoxy − benzoquinone

A 580

mM NADH

FIG. 9

EP 0 279 988 B1

COLOR THRESHOLD CONTROL

Benzoquinone

FIG. IO

EP 0 279 988 B1

## COLOR THRESHOLD CONTROL & ETHANOL

Benzoquinone

FIG. 11

EP 0 279 988 B1

EP 0 279 988 B1

FIG. 13

A + B = C

COLOR IS LINEARLY RELATED TO NADH OVER A RANGE
FROM 0 M NADH TO A POINT WHERE EITHER THE
CHROMOGEN OR THE SECOND SUBSTRATE IS EXHAUSTED

FIG. 12

FIG. 14

CAPILLARY MEASURING AVE

| | | |
|---|---|---|
| A | DEHYDROGENASE | |
| B | pH SEPARATING LAYER | DIFFERENT pH LEVELS |
| C | CCAC LAYER | |

EP 0 279 988 B1